# NEW EUROPEAN PATENT SPECIFICATION

(11) **EP 0 652 772 B2**
(45) Date of publication and mention of the opposition decision: **23.07.2008**
(45) Mention of the grant of the patent: 20.11.2002
(21) Application number: 93918571.6
(22) Date of filing: 02.08.1993
(51) Int. Cl.: A61K 39/12, A61K 39/245

(54) **HERPESVIRUS VACCINES**
HERPESVIRUS IMPFSTOFFE
VACCIN CONTRE L'HERPESVIRUS

(30) Priority: 31.07.1992 US 922912
(43) Date of publication of application: 17.05.1995
(73) Proprietor: The President and Fellows of Harvard College, Cambridge, MA 02138 (US)
(72) Inventor: KNIPE, David, Auburndale, MA 02115 (US)
(74) Representative: Kirkham, Nicholas Andrew
(86) International application number: PCT/US1993/007245
(87) International publication number: WO 1994/003207

(56) References cited:
- EP-A- 0 453 242
- WO-A-92/05263
- US-A- 4 859 587
- JOURNAL OF VIROLOGY, vol. 64, no. 4, April 1990 pages 1704-1715, RICE ET AL 'GENETIC EVIDENCE FOR TWO DISTINCT TRANSACTIVATION FUNCTIONS OF THE HERPES SIMPLEX VIRUS ALPHA PROTEIN ICP27'
- JOURNAL OF GENERAL VIROLOGY, vol. 73, no. 4, April 1992 pages 967-970, ROBERTSON ET AL 'PERIPHERAL REPLICATION AND LATENCY REACTIVATION KINETICS OF THE NON-NEUROVIRULENT HERPES SIMPLEX VIRUS TYPE 1 VARIANT 1716'
- JOURNAL OF GENERAL VIROLOGY, vol. 73, no. 5, May 1992 pages 1281-1285, SPECK ET AL 'SYNCHRONOUS APPEARANCE OF ANTIGEN-POSITIVE AND LATENTLY INFECTED NEURONS IN SPINAL GANGLIA OF MICE INFECTED WITH A VIRULENT STRAIN OF HERPES SIMPLEX VIRUS'
- JOURNAL OF VIROLOGY, vol. 66, no. 12, December 1992 pages 7067-7072, NGUYEN ET AL 'REPLICATION-DEFECTIVE MUTANTS OF HERPES SIMPLEX VIRUS (HSV) INDUCE CELLULAR IMMUNITY AND PROTECT AGAINST LETHAL HSV INFECTION'
- Journal of Virology, Vol. 45, No. 1, issued January 1983, WELLER et al., "Genetic Anylsis of Temperature-Sensitive Mutants Which Define the Gene for the Major Herpes Simplex Virus Type 1 DNA-Binding Protein", pages 354-366, especially pages 356-357.
- Journal of Virology, Vol. 63, No. 12, issued December 1989, GAO et al., "Genetic Evidence for Multiple Nuclear Functions of the Herpes Simplex Virus ICP8 DNA-Binding Protein", pages 5258-5267, see entire article.

## Description

### Background of the Invention

The field of the invention is herpesvirus vaccines.

Herpesviruses are enveloped double stranded DNA-containing viruses in an icosahedral nucleocapsid. At least seven herpesviruses are associated with disease in humans, including herpes simplex virus type 1 (HSV-1), herpes simplex virus type 2 (HSV-2), varicella zoster virus (VZV), Epstein Barr virus (EBV), cytomegalovirus (CMV), human herpesvirus 6 (HHV-6) and human herpesvirus-7 (HHV-7). In general, a common property of herpesviruses is their ability to establish a latent infection in the host in which they multiply.

HSV-1 is one of the most intensively studied herpesviruses. HSV-1 exhibits a pattern of gene expression during productive infection which is stringently regulated (reviewed in Fields and Knipe, Virology, 1990, Raven Press, NY). The 70 to 80 genes encoded by this virus are classified in part according to the kinetics of their expression. Expression of each class of genes is dependent upon expression of genes from the preceding class. The viral α or immediate early genes are expressed first, followed by the viral β or early genes which in turn are followed by the γ or late genes. The γ genes are further subdivided into γ-1 and γ-2 genes depending upon the extent to which their expression relies upon viral DNA replication.

Several viral proteins have been shown to regulate expression of HSV-1 genes. The ICP4 gene product is essential for β and γ gene expression (DeLuca et al., 1985, J. Virol. 56:558). The ICP27 gene product is required for γ gene expression and for viral DNA replication (McCarthy et al.,1989, J. Virol. 63:18). The major DNA-binding protein (ICP8), a β gene product, is also required for viral DNA replication and for γ gene expression (Gao and Knipe, 1989, J. Virol. 63:5258; Quinlan et al., 1984, Cell 36:657).

Diseases caused by herpesviruses in humans vary from mild to severe, and in some cases, infection with these viruses is life-threatening.

Vaccination is a common approach to prevention of disease. Various vaccines based on isolated immunogens, and on live, attenuated virus have been proposed for herpesviruses: HSV -- Roizman, U.S. Patent 4,859,587; and Meignier et al. (1988) J. Inf. Dis. 3: 603-613. VZV -- Takahasi et al. (1975) Biken J. 18:25-33. CMV -- Elek & Stern (1974) Lancet 1:1-5; and Plotkin et al. (1975) Infect. Immun 12:521-527.

### Summary of the Invention

The invention features a herpesvirus vaccine according to claim 1. Specifically, the virus is live in the sense that it generally retains the ability to infect target cells in the host to be protected. Infection will not produce progeny, yet the virus elicits a protective immune repsonse, e.g., via virally induced or encoded immunogens produced by infected cells. Protection means that the host mounts an immune response to the vaccine so that subsequent infection by wild-type virus is prevented or is less severe in terms of duration and extent. Particularly, establishment of latent infection is prevented. Standard animal tests can be used to verify protection. For HSV, for example, mouse and rabbit models can be used to establish protective ability. Coen et al. Proc. Nat'l. Acad. Sci. (1989) 86: 4736-3740. Standard mouse footpad models guinea pig vaginal model can be used for HSV-2.

WO 92/05263 discloses a mutant virus for use as a vaccine but in contrast to the present invention the viruses disclosed in WO92/05263 have lost the capacity to infect other cells due to a changed virion surface.

The herpesvirus is HSV-1, HSV-2, VZV, EBV, HHV-6 or HHV-7. The mutation is in the gene encoding HSV-1 ICP27 or HSV-1 ICP8, or the corresponding homologs of those genes in a non-HSV-1 herpesvirus. A preferred mutant herpesvirus for use in the vaccine is n504R or d301. More preferably, the herpesvirus contains a mutation in both the HSV-1 ICP27, or ICP8 genes or in both the corresponding homolog genes of a non-HSV-1 herpesvirus. The mutant herpesvirus may also be engineered to include one or more heterologous genes so as to be a vaccine expression vector which induces protection against a pathogen unrelated to the parent herpesvirus. Preferably, the mutant herpesvirus includes the wild type form of the viral thymidine kinase gene.

A second aspect of the invention features methods of making a herpesvirus vaccine by constructing the above-described mutated herpesvirus and suspending it in a pharmaceutically acceptable carrier.

Other aspects of the invention include immunizing a human against a herpesvirus by administering the above-described mutated herpesvirus vaccine.

### Detailed Description

The drawings are first described.

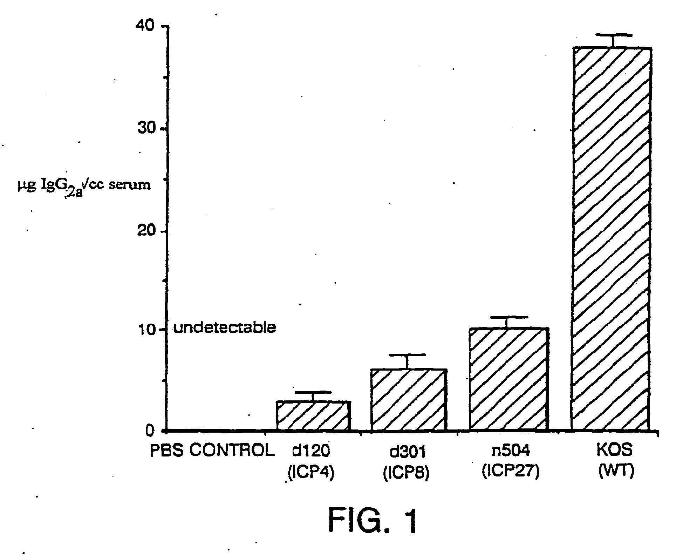

Figure 1 is a graphical representation of the antibody response in mice inoculated with wild type HSV-1 or with the replication deficient mutants according to the invention: d301 and n504; results with a third mutant (d120) not covered by the invention are also disclosed. Balb/c mice (8 per group) were challenged intraperitoneally with 10⁶ plaque forming units (pfu) of either wild type HSV-1 or mutants d120, d301 or n504. Two weeks post-inoculation, the amount of HSV-1 specific IgG₂ₐ present in the serum obtained from these mice was measured by ELISA. The data are presented as the mean with the standard error of the mean. This experiment presents data which is typical of a total of four experiments.

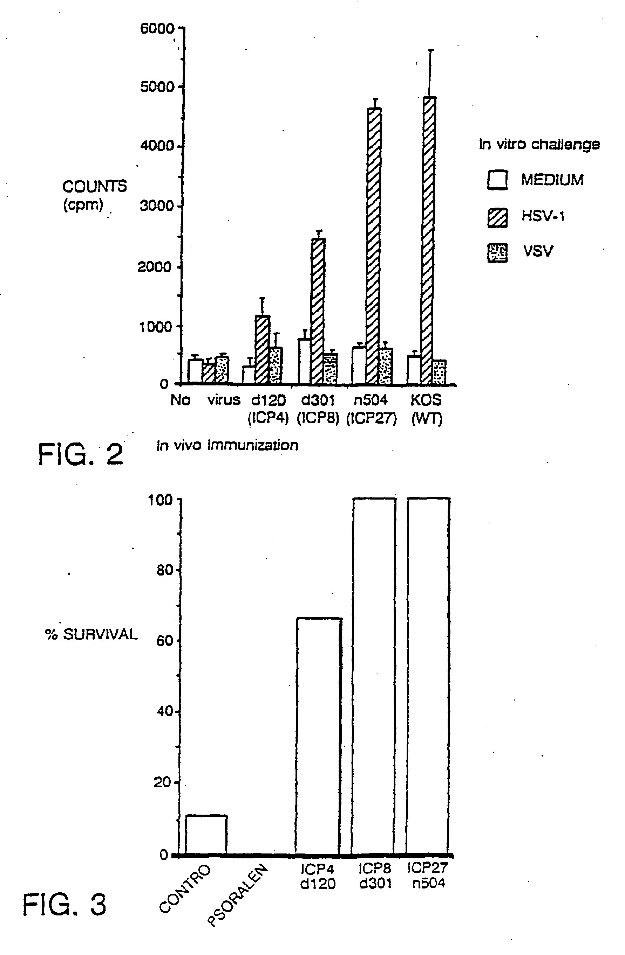

Figure 2 is a graphical representation of the T cell response in mice inoculated with wild type virus or with the replication deficient mutants d120, d301 or n504. T cells obtained from mice inoculated with each virus were incubated in the presence of UV irradiated USV-1 or with vesicular stomatitis virus (VSV) at a multiplicity of infection of 1 pfu per cell. T cells obtained from nonimmunized mice served as a negative control. The data are presented as the mean with the standard error of the mean. This experiment is representative of a total of three experiments.

Figure 3 is a graphical representation of the survival of mice inoculated with replication deficient mutants that were subsequently challenged with wild type virus. Groups of mice were inoculated intraperitoneally with 10⁶ pfu of either d120 (6 mice), d301 (6 mice), n504 (6 mice) or an equivalent dose of psoralen-inactivated wild type HSV-1 (5 mice). Control mice (9 mice) were injected intraperitoneally with phosphate buffered saline (PBS). Six weeks post-inoculation, all the mice were challenged with 5 x 10⁷ pfu of HSV-1 (strain mp) intraperitoneally. Most of the mice died between day 7 and day 11 post-challenge. Survival rates were recorded for 4 weeks post-challenge. This experiment is representative of a total of four experiments.

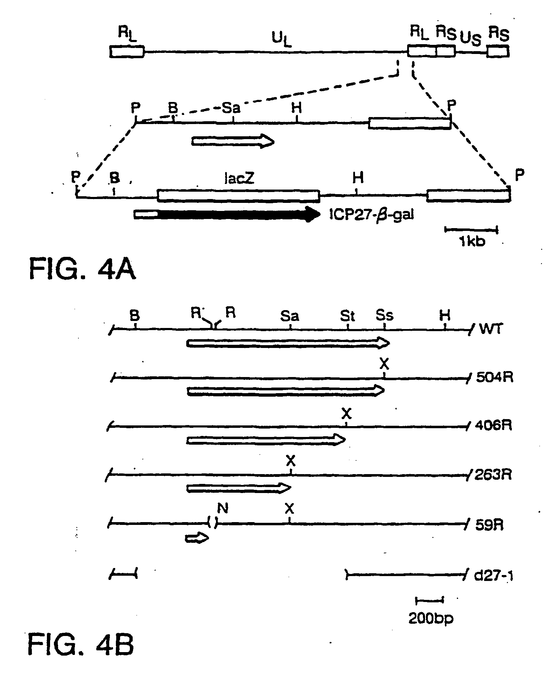

Figure 4 is a diagrammatic representation of the location and structure of wild type and mutant HSV-1 ICP27 genes. (A) Structures of the wild type and the *lacZ* insertion mutant genes. A representation of the prototype arrangement of the HSV-1 genome is shown at the top. A PstI restriction fragment from the wild type and the d27-*lacZ1* genome is shown below. The narrow lines denote unique (U) regions of the viral genome, the open bars denote repeat regions (R), and the hatched bar denotes *E*.*coli lacZ* sequences. The upper arrow represents the coding sequences for the 63 kDa ICP27 protein and the bottom arrow represents the coding sequences for the approximately 137 kDa ICP27-β-galactosidase fusion protein. (B) Structures of the wild type, nonsense and deletion mutant genes. ICP27 mutant genes were constructed by deleting restriction fragments (parentheses) or inserting XbaI or NheI oligonucleotide linkers (X and N, respectively) containing stop codons in all three reading frames. The arrows represent either the wild type ICP27 protein (top) or the truncated forms of ICP27 encoded by the nonsense mutants. Restriction sites : P, PstI; B, BamHI; Sa, SalI; H HpaI; R, RsrII; St, StuI; Ss, SspI; X, XbaI; N, NheI.

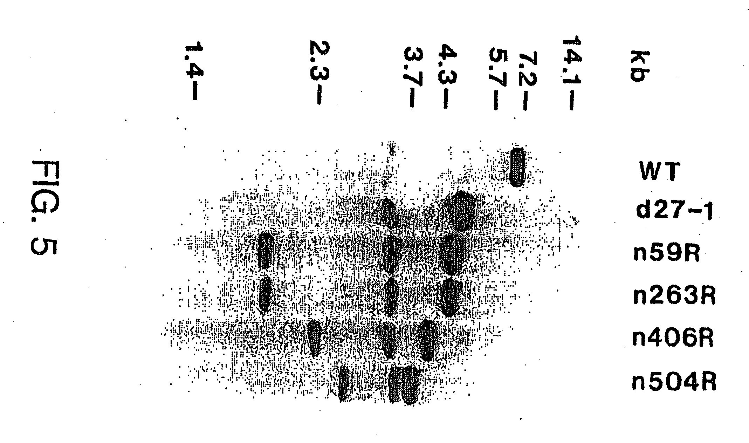

Figure 5 is an autoradiogram of Southern blot hybridization analysis of HSV-1 ICP27 mutant genomes. Purified viral DNAs were digested with PstI and XbaI, subjected to electrophoresis in an agarose gel and transferred to a nylon filter. The filter was probed with ³²P-labeled DNA from a plasmid which contains a 6.1 kb PstI insert from the region of the HSV-1 genome encoding ICP27. The numbers on the left of the figure are the relative positions of standard DNA size markers.

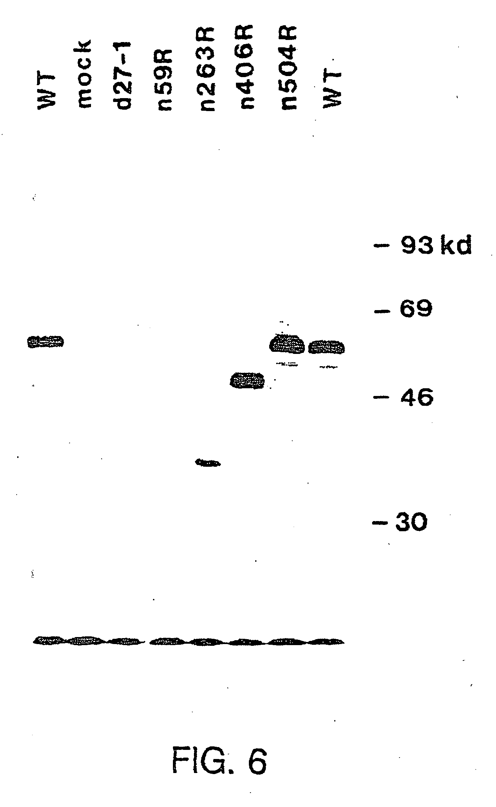

Figure 6 is a picture of western blot analysis of ICP27-related polypeptides expressed in mutant virus infected cells. Total protein was prepared from infected Vero cells at 10 hours (h) post infection (PI), separated by SDS-PAGE and electrophoretically transferred to a nitrocellulose filter. The filter was probed with H1113. The numbers at the right of the figure are the relative positions of standard protein size markers.

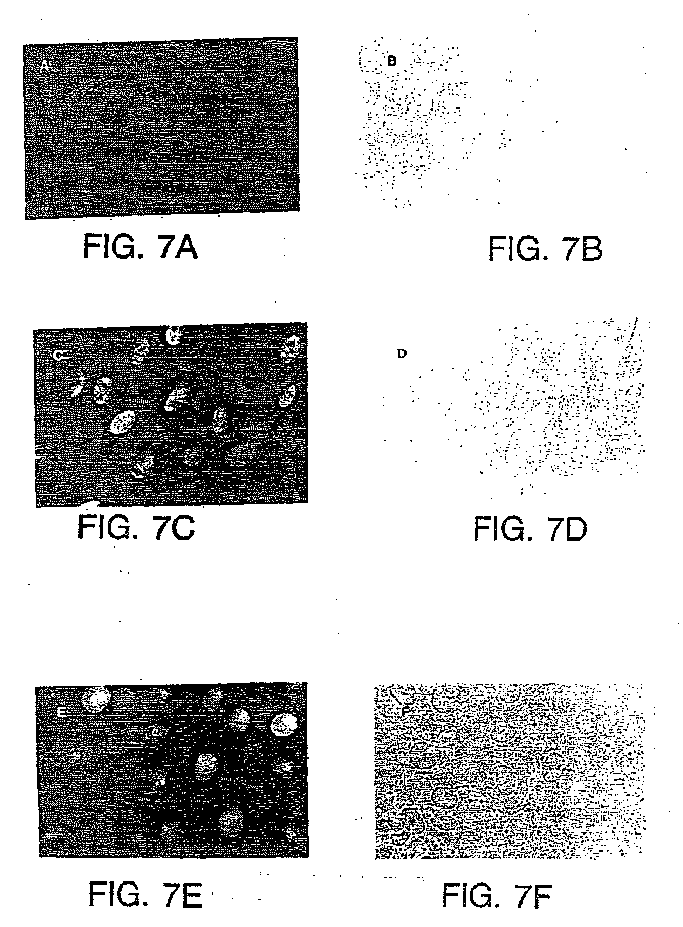

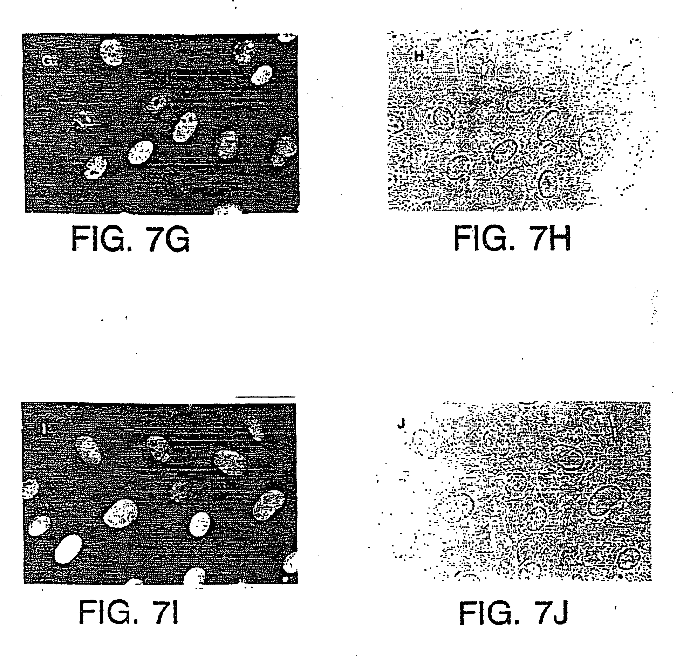

Figure 7 is a collection of photomicrographs depicting the intracellular localization of mutant ICP27 molecules. Vero cells were mock infected (A and B) or infected with wild type virus (C and D), n263R (E and F), n406R (G and H), or n504R (I and J). At 4 h PI, the cells were fixed and processed for immunofluorescence microscopy using the antibody H1113. Panels A, C, E, G and I are immunofluorescence micrographs and panels B, D, F, H and J are the corresponding phase contrast micrographs.

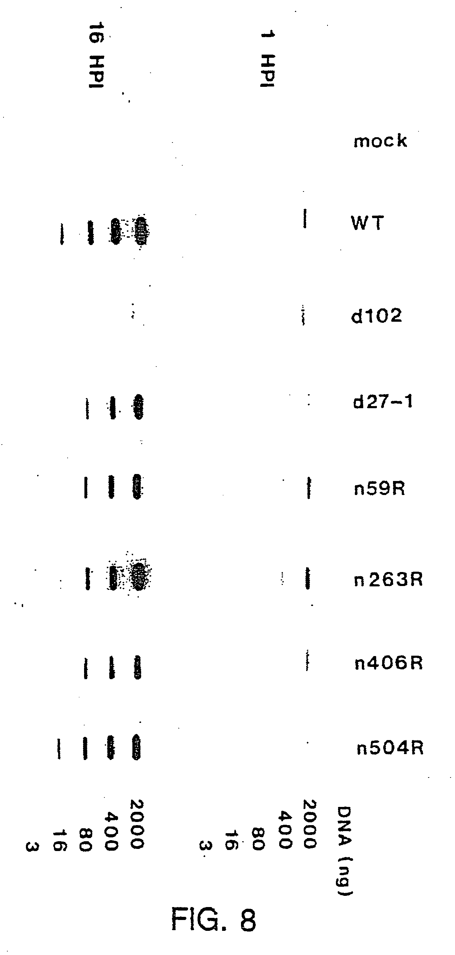

Figure 8 is a collection of autoradiograms depicting the ability of ICP27 mutants to replicate viral DNA. Vero cells were mock infected or infected with wild type HSV-1 or various mutants. Total cell DNA was prepared immediately after virus adsorption (1 h PI) or near the end of the infectious cycle (16 h PI). Equal amount of each DNA sample were subjected to five-fold serial dilutions, and the dilutions were applied to a nitrocellulose filter using a slot blot manifold. The filter was probed with ³²P-labeled DNA from pSHZ (Nabel et al. (1988) Science, cited elsewhere).

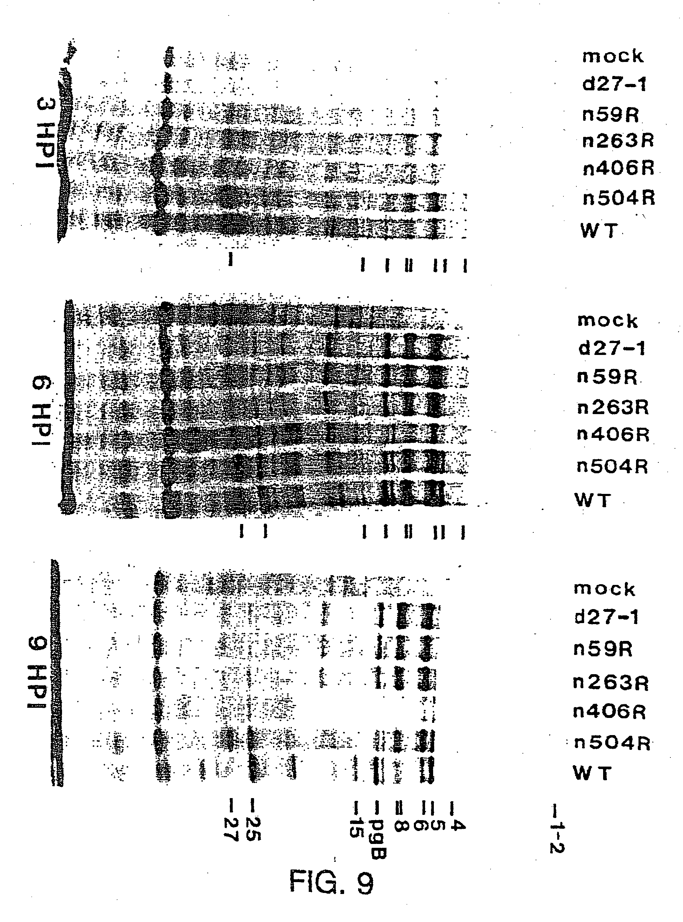

Figure 9 is an autoradiogram depicting protein synthesis in ICP27 mutant infected cells. Vero cells were mock infected or infected with wild type HSV-1 or various ICP27 mutants. At 3, 6 or 9 h PI, the cells were labeled for 30 minutes with [³⁵S]-methionine and then harvested. Proteins were extracted from the lysates and equal amounts of protein were subjected to SDS-PAGE and autoradiography. The relative positions of various HSV-1 proteins is shown at the right of each panel.

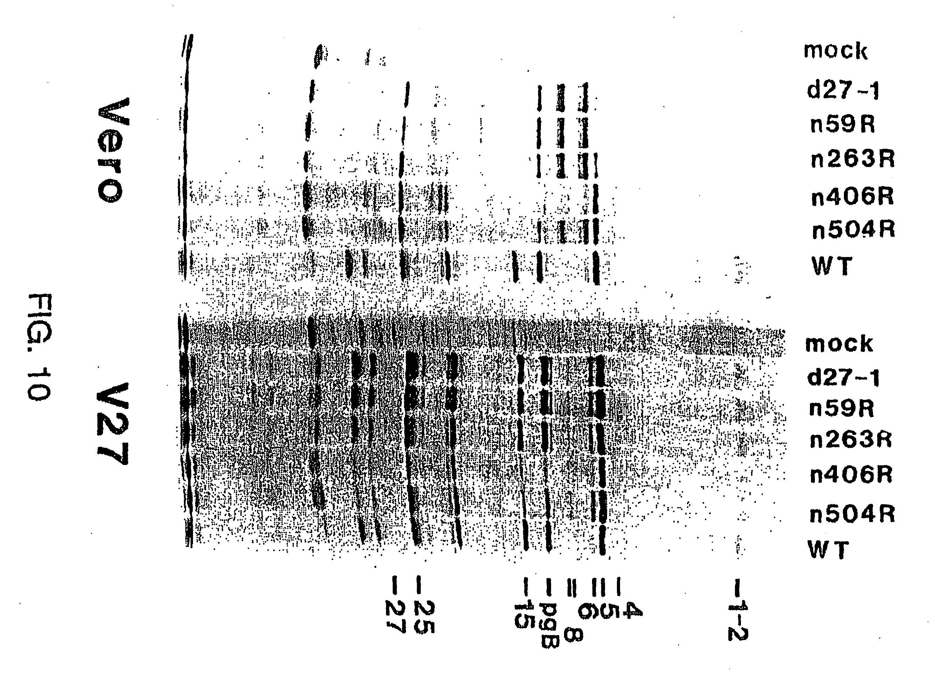

Figure 10 is a autoradiogram depicting complementation of aberrant protein synthesis by growth of ICP27 mutants in V27 cells. Vero or V27 cells were mock infected or infected wild type HSV-1 or various ICP27 mutants. Cells were labeled for 30 minutes at 15 h PI with [³⁵S]-methionine and protein synthesis was analyzed as described for Figure 9. The positions of several HSV-1 proteins are shown to the right of the figure.

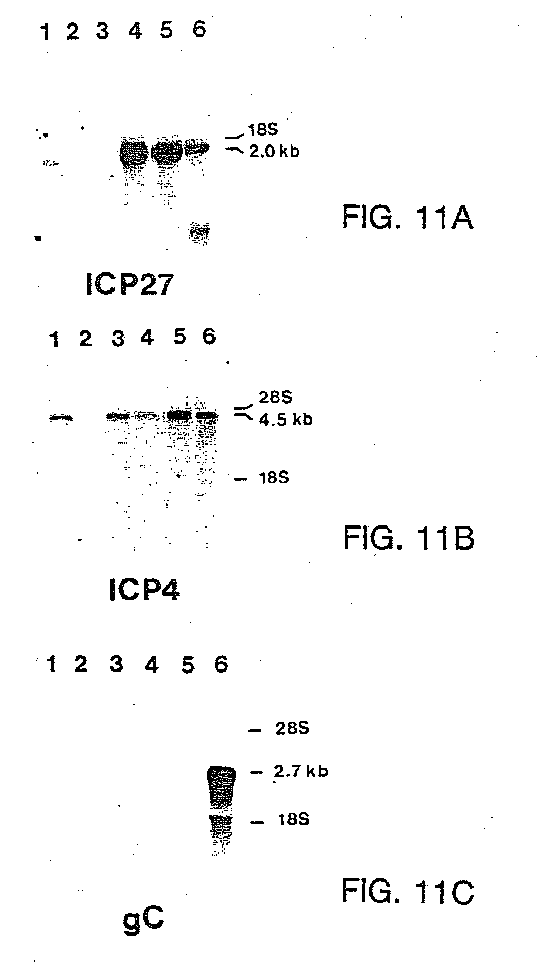

Figure 11 is an autoradiogram depicting accumulation of viral mRNAs in ICP27 mutant infected cells. Vero cells were mock infected or infected with wild type HSV-1 or various ICP27 mutants. At 9 h PI, cytoplasmic RNA was prepared. Equal amounts of RNA were subjected to Northern blot hybridization analysis using ³²P-labeled probes specific for ICP27 (A), ICP4 (B), or gC (C) mRNAs. The lanes contain RNA from mock infected cells (lanes 2) or cells infected with wild type HSV-1 in the presence of 400 µg/ml of phosphonoacetic acid (lanes 1), d27-1 (lanes 3), n59R (lanes 4), n504R (lanes 5), or wild type HSV-1 (lanes 6). The relative positions of viral and ribosomal RNAs are shown to the right of each panel.

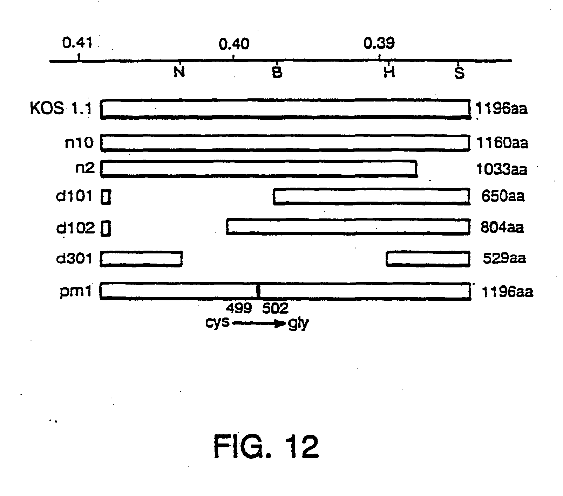

Figure 12 is a diagrammatic representation of the locations of the ICP8 nonsense (n), deletion (d) and point (pm) mutations. The location of the ICP8 coding region on the HSV-1 genome is shown at the top of the figure. The restriction sites shown are BamHI (B), NotI (N) and Sail (S).

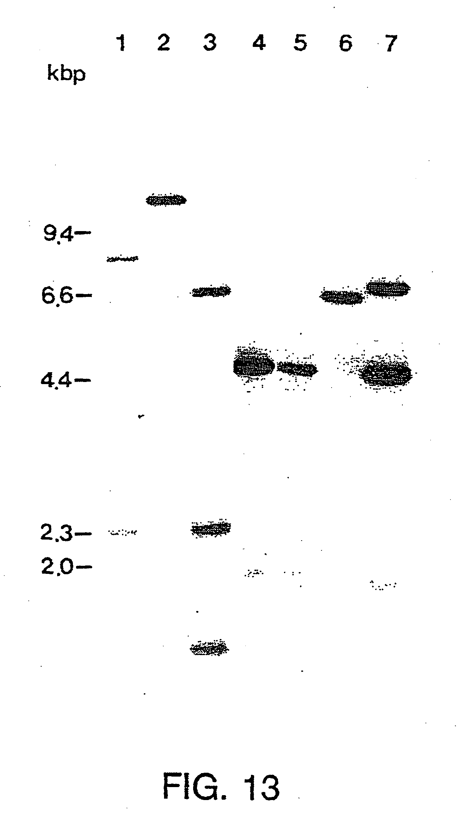

Figure 13 is an autoradiogram of Southern blot hybridization analysis of wild type HSV-1, HD-2 and n2 DNAs. DNA obtained from cells infected with wild type HSV-1, HD-2 and n2 was digested with BamHI-XbaI (lanes 1, 2 and 3) and KpnI (lanes 4, 5 and 6) and subjected to electrophoresis in parallel with KpnI digested pSG18-SacI (lane 7). The DNA was transferred to a nitrocellulose filter and hybridized to ³²P-labeled plasmid pICP8. The relative positions of standard size markers are at the left of the figure.

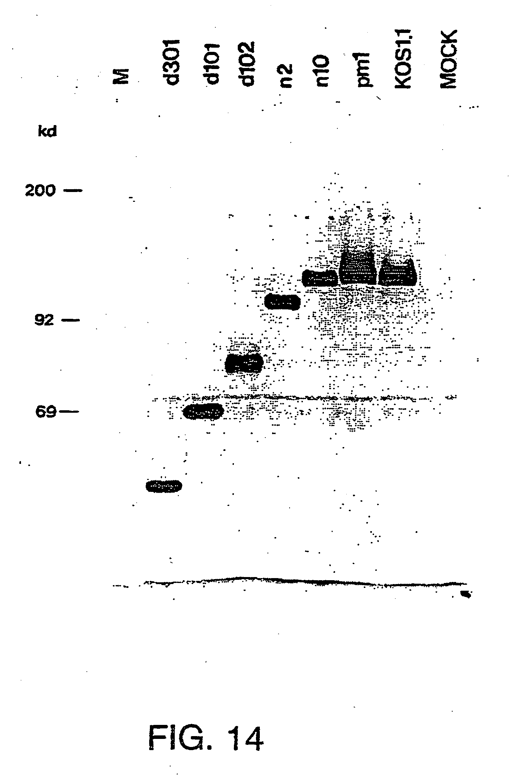

Figure 14 is an autoradiogram of western blot analysis of ICP8 specific polypeptides obtained from mutant infected cells. Vero cells were infected with each virus in the presence of phosphonoacetic acid and were harvested at 6 h PI. Proteins in the cellular extracts were separated by SDS-PAGE and electrophoretically transferred to nitrocellulose. The filter was probed with polyclonal rabbit antiserum to ICP8. The relative positions of standard size markers are to the left of the figure.

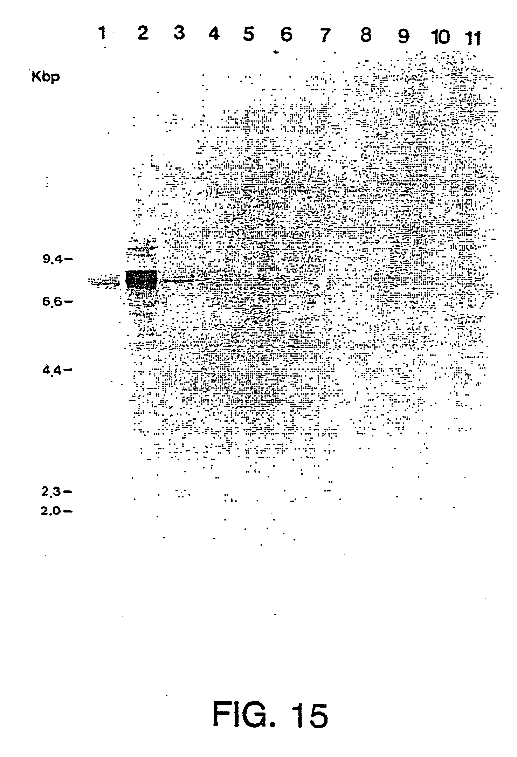

Figure 15 is a fluorogram of analysis of viral DNA replication. Vero cells were mock infected (lane 1) or infected with wild type virus in the absence of phosphonoacetic acid (lanes 2 - 4), or in the presence of phosphonoacetic acid (lane 5), pm1 (lane 6), n10 (lane 7), n2 (lane 8), d102 (lane 9), d101 (lane 10), or d301 (lane 11) and were labeled with [³H]-thymidine from 6 to 10 h PI. Total DNA was isolated and 10 µg of each sample (except lane 3 [5 µg] and lane 4 [1 µg]) was digested with BamHI and XhoI and separated by agarose gel electrophoresis. After electrophoresis, the gel was treated with 1.0 M sodium salicylate for fluorography.

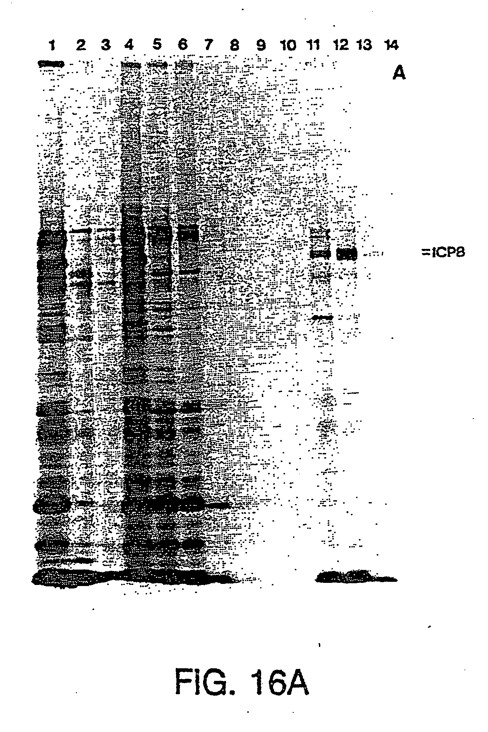

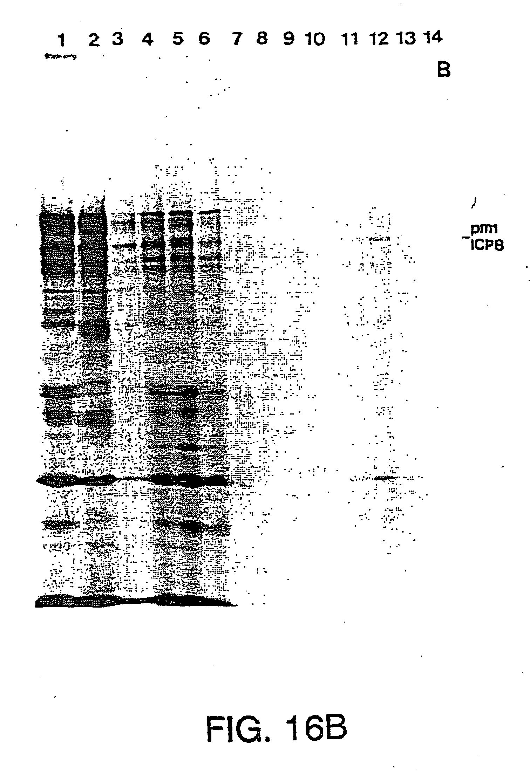

Figure 16 depicts binding of ICP8 to single stranded DNA cellulose. Vero cells were infected with either wild type (A) or pm1 (B) in the presence of phosphonoacetic acid and were labeled with [³⁵S]-methionine from 4 to 6 h PI. Various protein fractions resolved on single stranded DNA cellulose columns were subjected to SDS-PAGE. Lanes: 1, total cellular lysate; 2, pellet from high salt DNase extraction; 3, pellet after dialysis; 4, extract loaded onto the column; 5 to 10, flowthrough and wash; 11, 0.3 M NaCl eluate; 12, 0.5 M NaCl eluate; 13, 1.0 M NaCl eluate; 14, 4.0 M NaCl eluate. The relative position of either wild type or mutated ICP8 is indicated to the right of each panel.

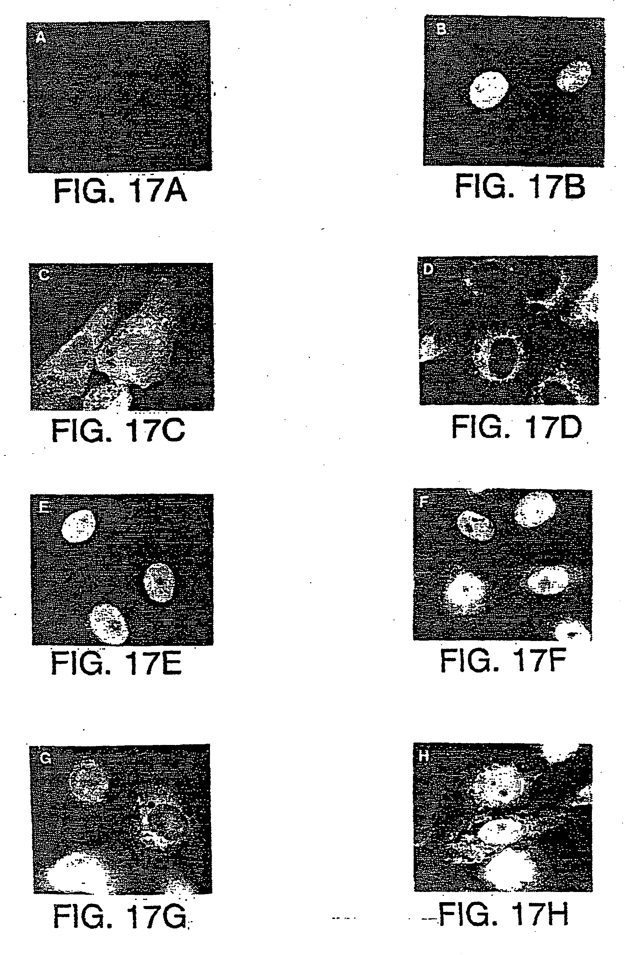

Figure 17 is a collection of photomicrographs depicting the subcellular localization of mutant ICP8 polypeptides. Vero cells were infected with wild type or ICP8 mutant viruses. At 4 h PI, the cells were fixed, permeabilized and incubated with either 793 anti-ICP8 monoclonal antibody and rhodamine-conjugated goat anti-mouse immunoglobulin (panels A through F and H), or anti-ICSP 11/12 polyclonal serum and fluorescein-conjugated goat anti-rabbit immunoglobulin (panel G). Immunofluorescence micrographs: A, mock-infected cells; B, wild-type infected cells in the presence of phosphonoacetic acid; C, n10-infected cells; D, d102-infected cells; E, pm1-infected cells; F, d301-infected cells; G, n2-infected cells; H, d101-infected cells.

### I. GENERATION OF VIRAL MUTANTS USEFUL AS VACCINES

In accordance with the invention, herpesvirus mutants useful as potential vaccine candidates can be constructed and tested using methods similar to those described below for specific mutants. Construction of such mutants is facilitated by the fact that the complete DNA sequences of four human herpesviruses, HSV-1, VZV, EBV, are known (McGeoch et al., 1988, J. Gen. Virol. 69:1531; McGeoch et al., 1985, J. Mol. Biol. 181:1; McGeoch et al., 1986, Nucl. Acids Res. 14:1727; Davison et al., 1986, J. Gen. Virol. 67:1759; Baer et al., 1984, Nature 310:207; Chee et al., 1990, Current Topics in Microbiol. and Immunol. 154:125), and restriction enzyme maps, partial sequence and the precise location of many of the genes in the remaining human herpesviruses are also known (Fields and Knipe, 1990, *Supra*). Furthermore, genomic libraries are available and a large volume of plasmids encoding many different herpesvirus specific genes are also available. See generally, Fields and Knipe Virology, 1990, Raven Press, N.Y., and references cited therein.

In general, methods for obtaining mutant viruses useful as potential vaccine candidates involve the same steps as those described below for the generation of viral mutants in either the HSV-1 ICP27 or ICP8 genes. First plasmids are constructed which comprise viral DNA encoding the appropriate mutation, flanked by viral DNA that will undergo homologous recombination. Next, this DNA is cotransfected into animal cells with the genomic viral DNA into which the mutation is to be inserted. The mutation will be inserted into this parental genome by a process of homologous recombination as the viral DNA is replicated in these cells. Progeny virus are screened for the presence of the mutation using any combination of the techniques described below. For example, progeny virus can be selected for their ability to replicate only in a cell line expressing a wild type complementing copy of the mutated gene, providing the expression of that gene is essential for virus replication. These viruses can then be screened for example, by Southern blot hybridization, western blotting, immunofluorescence, expression of a specific mRNA species etc. Such techniques are standard in the art of molecular virology and are described in detail in the molecular cloning manual by Sambrook et al., cited below.

The general goal for a viral vaccine is to induce extended (even lifetime) protection from disease and to be free of both initial and long term side effects. The vaccine should induce both protective humoral antibodies and cell-mediated immunity. Live virus vaccines should be incapable of spreading from vaccinees to non-vaccinated individuals, and should not be capable of latent infection in the vaccinee.

Mutant herpesviruses such as those described herein generally satisfy these criteria.
Specifically, vaccine candidates according to the invention should have at least the following properties: they should be viable and yet be effectively incapable of producing viable progeny virus in the host into which they are introduced; and, they should be capable of inducing a protective immune response in that host. Thus, strains of HSV-1, such as those described above, which contain mutated ICP27 or ICP8 genes, are potential vaccine candidates. These strains can be further improved by constructing additional mutations within their genomes. For example, viruses can be generated which contain additional deletions in the genes encoding the proteins designated ICP27 or ICP8. These viruses can be constructed and tested as described below. Additional mutations will also result in stable viruses.

Those skilled in the art will understand that other suitable mutations in the ICP27 and/or ICP8 proteins are operative in the invention. The experiments provided below can be used to screen candidate viruses with such mutations. Preferred candidate mutations are those which are mutated in the indicated ICP8 and/or ICP27 domains and which retain the desired mutant phenotype as indicated by the experiments described below.

In a similar manner, viruses containing mutations in more than one viral gene can be generated. These viruses may also have significant advantages in terms of safety and inability to spread over virus strains which are mutated in only one viral gene. Examples of these viruses include, but are not limited to, strains in which a mutation in the ICP8 gene is introduced into the genome of a virus which contains an existing mutation in the ICP27 gene. Conversely, a virus strain can be constructed in which a mutation in the ICP27 gene is introduced into the genome of a virus which contains an existing mutation in the ICP8 gene. In order to replicate such viruses, a cell line is generated which is capable of expressing both the wild type ICP27 and ICP8 genes. The procedures for the generation of cell lines which express more than one herpesvirus gene are well known in the art and are similar to those described above, recognizing that transfected cells take up each of the genes included in the transfection mixture. See, generally, Quinlan and Knipe, (1983) Mol. Cell. Biol. 5: 957-963.

The majority of virus-specific products responsible for eliciting a protective immune response are proteins and glycoproteins which are expressed in the infected cell and generally can also be found on the surface of the virion. In the case of the herpesviruses, some of the major antigenic determinants are glycoproteins encoded by the viral genome. Vaccine candidate strains of any herpesvirus can be constructed which are capable of expressing either one or more glycoproteins normally encoded by a different herpesvirus. For example, mutant strains of HSV-1 (containing mutations in the genes for either or both ICP27 and ICP8) can be generated which encode and are capable of expressing one or more glycoproteins normally encoded by HSV-2 or any of the other human herpesviruses. Such viruses are constructed using ordinary molecular biology techniques and the procedures described above.

One such example is now briefly described below. A gene or several genes encoding HSV-2 specific glycoproteins can be flanked on either side with approximately 100 - 300 bp of HSV-1 specific DNA, for example, HSV-1 specific thymidine kinase or more preferably, glycoprotein C DNA, or any other region of the HSV-1 genome that is not absolutely required for replication of the virus. It may be beneficial to retain an intact copy of the thymidine kinase gene in a vaccine strain because the product of this gene is required for activation of many anti-HSV compounds (Fields and Knipe, cited elsewhere). This hybrid DNA is cotransfected into cells with infectious HSV-1 specific DNA which contains a mutation in either or both the ICP8 and ICP27 genes. Progeny virus are then obtained and screened for the insertion of the HSV-2 gene(s) into the specific HSV-1 locus determined by the flanking sequences as described above. These viruses can then be assessed for their ability to elicit a protective immune response as described below. Such viruses can be useful in protecting individuals against both HSV-1 and HSV-2 because they may be capable of expressing antigenic determinants specific for both viruses. In a similar manner, genes encoding antigens normally expressed by other human herpesviruses, or other infectious agents including viruses, bacteria, fungi and parasites, can be recombined into the genetic background of a replication defective HSV-1, in order to obtain multiple protection from a single vaccination.

HSV-2 is known to encode many genes whose DNA sequence and protein products are homologous to those encoded by HSV-1 (Field and Knipe, 1990, cited elsewhere). In fact, HSV-2 encodes genes with very similar DNA sequence and to the HSV-1 ICP27 and ICP8 genes. The protein products of these HSV-2 ICP27 and ICP8 homologs have properties that are also very similar to their HSV-1 counterparts. See, Morse et al. (1978) J. Virol. 26:389-410; Marsden et al. (1978) J. Virol. 28:624-642. Thus, mutant strains of HSV-2 can be generated in a manner identical to that described for HSV-1 which contain mutations in either one or both of these genes. Such mutants are also vaccine candidates for HSV-2. Furthermore, genes encoding glycoproteins specific for other herpesviruses or infectious agents can be inserted into the genetic background of these mutant strains so that each strain is capable of expressing these heterologous genes in addition to HSV-2 glycoproteins.

The HSV-2 genome contains along its length two distinct regions of DNA that have been shown to be capable of transforming cells in tissue culture. These regions are termed *mtrII* and *mtrIII*, and their precise location on the HSV-2 genome is known (Galloway et al., 1984, Proc. Natl. Acad. Sci. USA 81:4736; Ali et al., Proc. Natl. Acad. Sci. USA 88:8257). In order to eliminate the possibility that vaccine strains of HSV-2 might be capable of transforming cells *in vivo*, these regions of DNA can be replaced by non-transforming HSV-1 sequences by homologous recombination. Replacement of such sequences is accomplished using procedures similar to those used in the generation of alterations in specific HSV-1 genes described below.

In addition to HSV-1 and HSV-2, other herpesviruses encode genes which are highly homologous to either the HSV-1 ICP27 or ICP8 genes. Viruses which encode HSV-1 ICP27 homologs include VZV, EBV, and the non-human equine herpesvirus type 1 (Davison et al., 1986, J. Gen. Virol. 67:1759; Baer et al., 1984, Nature 310:207; Holden et al., 1992, J. Virol. 66:664); and viruses which encode HSV-1 ICP8 homologs include VZV and EBV (Davison et al., 1986, J. Gen. Virol. 67:1759; Baer et al., 1984, Nature 310:207; Chee et al., 1990, Current Topics in Microbiol. and Immunol. 154:125). It is therefore possible, using the methods described above, to generate candidate vaccine strains of these viruses which may have properties similar to those described above for HSV-1 strains containing mutations in the genes encoding either ICP27 or ICP8.

The vaccines of the invention are limited to herpesviruses containing mutations in the ICP27 or ICP8 genes or their respective homologs.

For example, glycoprotein immunogens are disclosed by Sarmiento et al (1979) J. VIrol. 29:1159 ("gB"); Coker et al. (1978) J. Virol. 47:172-181 ("gD"); and DeSai et al. (1988) J. Gen. Virol. 69:1147-1156 ("gH"). These glycoprotein immunogens may be inserted in a mutated background, e.g., ICP27 or ICP8. Similarly, capsid protein mutants may be useful.

### Administration and Dosage:

Those skilled in the art will understand that dosage can be optimized using standard procedures. In general, the vaccines are formulated in suitable sterilized buffer and administered (e.g., by subcutaneous, intramuscular or intradermal injection) at a dosage of between 10³ and 10⁹ PFU/kg.

### SPECIFIC EXAMPLES OF REPLICATION DEFICIENT MUTANTS OF HSV-1 INDUCE CELLULAR IMMUNITY AND PROTECT AGAINST LETHAL INFECTION

The following examples are provided to illustrate the invention, not to limit it. Those skilled in the art will understand that the specific constructions provided below may be changed in numerous ways, consistent with the above described invention while retaining the critical properties of the vaccine.

### Methods

The following materials and methods were used in the examples described below.
**Mice.** Female BALB/c mice were purchased from Taconic Laboratory, Germantown, NY and were used at 6 to 12 weeks of age. Mice were injected intraperitoneally with 0.5 ml of PBS or with 0.5 ml of virus suspended in PBS.
**Viruses.** The HSV-1 wild-type strain KOS 1.1 and strain mP were propagated and assayed on Vero cells as described (Quinlan and Knipe, 1985, Mol. Cell. Biol. 5:957). A virus containing a mutation in the gene encoding ICP8, termed d301, was generated as described by Gao et al. cited elsewhere. A virus containing a mutation in the ICP27 gene, termed n504R, was generated as described below. A virus (d120) encoding a mutated ICP4 gene was generated as described by DeLuca et al. (1985, J. Virol. 56:558). VSV was propagated as described by Horn et al. (1989, J. Virol. 63:4157). All virus stocks were stored at -70°C and a newly thawed aliquot from each stock was used in each experiment. Preparations of UV-irradiated HSV-1 and VSV were obtained by irradiating each virus at 0°C using a 30-W UV source (G30T8; General Electric) for 45 minutes at a distance of 5 cm. Psoralen inactivated virus preparations were generated by Lee Biomolecular (San Diego, CA).
**Assays for T cell activity.** Immune spleen cells were obtained from mice which had been inoculated intraperitoneally 3-4 weeks earlier with 10⁶ pfu of wild type HSV-1, or with viral mutants containing mutations in the genes for either ICP4, ICP27 or ICPB. Mice which were inoculated with PBS served as negative controls. Spleen cells obtained from such mice were depleted of erythrocyte and polymorphonuclear leukocytes by Ficoll (Registered Trade Mark)-hypaque gradient sedimentation. B lymphocytes were removed from the mixture by incubating splenocytes with antibodies specific for B lymphocytes for 30 minutes at 4°C. The cells were then washed and incubated with goat anti-rat-antibody coated latex-polymer beads containing a magnetic core (Advanced Magnetics, Cambridge, MA). J11-d2 positive cells which bound to the magnetic beads were then removed using a magnet (BioMag Separator, Advanced Magnetics). The remaining cells in the mixture consisting of >95% T cells, were washed and incubated at a concentration of 10⁵ cells/well in a total volume of 0.2 ml in 96 well round-bottom culture plates (Nunc, Roskilde, Denmark). Samples of cells were plated in quadruplicate. Responder cells were stimulated with UV irradiated wild type HSV-1. Control cells to which virus was not added were prepared in parallel. The cells were incubated in Dulbecco's modified Eagle medium (Hazelton) supplemented with 5% bovine calf serum (Hyclone Labs; which serum had been inactivated at 56°C for 1 hour), 100 U/ml of penicillin (Gibco), 100 U/ml of streptomycin, 1 mM sodium pyruvate (Gibco), 0.1 mM nonessential amino acids (Gibco), 10⁻⁴ mM 2-mercaptoethanol (Sigma), and 2 mM-glutamine (Gibco). The cells were incubated for 3 days in the presence of 10% CO₂ at 37°C. [³H]-thymidine (New England Nuclear), at a concentration of 1 µCi/well, was added for 6 hours and the cells were harvested using a Skratron (Registered Trade Mark) Cell Harvester. The amount of radioactivity in each sample was determined using a liquid scintillation counter (Beta Trac 6895; TM Analytic).
**Antibody assays.** Samples of serum obtained from infected mice were analyzed for the presence of HSV-1 specific antibodies using an ELISA. The protocol used was identical to that described by Kahlon and Whitley (1988, J. Inf. Dis. 158:925) except that it was adapted for murine serum. Microtiter plates (Linbro/Titertek) were treated with 0.1 ml of a 1:50 dilution of 10⁷ pfu HSV-1 suspended in PBS overnight. Serum obtained from mice immunized with each virus as described above was obtained by retroorbital bleeding of the mice. Microplates coated with HSV-1 were washed three times and incubated with 100 µl of a 1:100 dilution of serum followed by a 1:3 dilution of the same serum overnight at room temperature. The microplates were washed again and incubated with goat anti-mouse IgG₂ alkaline phosphatase at a 1:250 dilution (Southern Biotechnology) for 3 hours at 37°C. Thirty minutes after the addition of 1 mg/ml of the substrate for alkaline phosphatase (Sigma 104), the reaction was stopped by the addition of 75 µl of 3 N NaOH. The results of the experiment were obtained using an ELISA reader at 405 nm. Pooled serum from wild type HSV-1 immunized mice served as a positive control and pooled naive mouse serum served as a negative control. Mouse sera were run individually, and the data are presented as mean and the standard errors of the mean.

### Results

### Use of replication defective viruses; lack of morbidity.

To examine whether replication-defective viruses (i.e., those containing mutations in the genes encoding either ICP8 or ICP27) were lethal when inoculated into mice, mice were injected with either live wild type HSV-1 or the mutant viruses d301 or n504. Mice which received as much as 10⁸ pfu of each mutant appeared healthy and were unaffected by the viruses. Littermates which received 10⁷ pfu of wild type HSV-1 all died.
**Induction of HSV-1 specific antibodies in mice inoculated with mutant viruses.** To determine whether the mutant viruses were capable of inducing HSV-1 specific antibodies in mice, serum was obtained from the mice two weeks after inoculation and examined for the presence of HSV-1 specific antibodies. Reproducibly, while HSV-1 specific antibodies could be detected in the sera of these mice, the levels of the antibodies were markedly lower than those in the sera of mice inoculated with wild type virus. A higher level of antibody was evident in sera obtained from mice inoculated with n504 than in the sera obtained from mice inoculated with d301. Similar results were obtained at both two and four weeks PI in a second independent experiment.

To determine whether expression of HSV-1 β proteins was necessary for induction of these antibodies, mice were inoculated with 10⁶ pfu of the ICP4 deletion mutant d120 which does not express either β or γ proteins. While levels of antibodies specific for HSV-1 above control levels could be detected in the sera of these mice, these levels were significantly below those observed in sera from mice inoculated with the ICP8 or ICP27 mutants (Figure 1).
**Induction of T cell response in mice inoculated with viral mutants.** To examine the ability of the mutants (each of which is deficient in the production of late viral gene products) to induce an HSV-1 specific T cell response, the response of splenic T cells obtained from inoculated mice to viral antigens was measured *in vitro*. Mice received 10⁶ pfu of either live wild type (KOS 1.1) virus or the replication defective mutants d120, d301 and n504. Three weeks later, T-cells obtained from mice in each group were incubated *in vitro* in the presence of UV-irradiated HSV strain mP. To control for the effect of non-specific T cell stimulation, the T cell response to an unrelated virus, VSV, was also evaluated. Splenic T cells from mice immunized with VSV did not proliferate in response to HSV-1, while the same cells did proliferate in response to VSV. Stimulation of T cell activity at levels above background was observed in splenic T cells obtained from mice inoculated with each of the replication deficient viruses (Figure 2). The results suggest that immunization of mice with the mutant viruses induced less stimulation of T cells than that induced following immunization with wild type virus.

Notwithstanding, these mutant viruses induced substantial T cell reactivity (Figure 2).
**Induction of protective immunity by replication-defective viruses.** To assess the ability of replication-defective viruses to induce protective immunity to lethal HSV-1, mice were inoculated with 10⁶ pfu of each of the replication deficient mutants. Three to six weeks later (depending on the experiment), all the mice were challenged with a lethal dose (5 X 10⁷ pfu) of a virulent strain of HSV-1 (mP). In three separate experiments, mice which had been inoculated with the mutants d301 or n504 were protected against challenge by the wild type virus, in that their survival rate was 100%. Control mice, which had received only the wild type virus had a survival rate of less than 20%. Reproducibly, in subsequent experiments performed with all three mutant viruses (d120, d301 and n504), while only 1/9 control (PBS injected) mice survived, preinoculation of mice with either the ICP27 or ICP8 mutants resulted in 100% survival following challenge with wild type virus. Even the ICP4 mutant (d120), which expresses only the immediate-early genes, protected the majority of mice. In contrast, UV irradiated virus had a minimal protective effect and immunization with psoralen-inactivated virus did not protect mice against lethal challenge (Figure 3).

In summary, replication-defective mutants of HSV-1 are capable of inducing both humoral and cellular immunity in mice inoculated with such viruses. Inoculation of mice with these mutants serves to protect these mice against challenge with a lethal dose of wild type HSV-1. Since cellular immunity is especially important in protection against infection with herpes simplex virus (Whitley, 1990, In: Virology, ed. Fields and Knipe, Raven Press, p 1843-1887), any agent capable of inducing such immunity is a potential vaccine candidate. Candidate vaccines such as those described above are also especially useful because they comprise viruses which are replication defective. Since these viruses cannot produce progeny viruses, they are substantially safer than conventional attenuated live virus vaccines. Mutant viruses such as those described above cannot replicate in cells which do not express a wild type complementing form of the gene. They therefore cannot spread beyond the site of the initial infection. An important implication of this observation for the pathogenesis of herpesviruses is that such mutant viruses are unlikely to be capable of establishing a latent infection in the host into which they are introduced. Consistent with this theory is the fact that no evidence for a latent infection in mice inoculated on the cornea with either d301 or n504 could be detected when trigeminal ganglia obtained from these mice were examined by *in situ* hybridization for latency-associated transcription and expression. See generally, Coen et al. Proc. Natl. Acad. Sci. USA 86:4736 et seq. (1989).

Construction and characterization of viral mutants such as those used in the study described above, and of additional mutants useful as candidate viral vaccines is now described below.

### CONSTRUCTION AND CHARACTERIZATION OF VIRUSES ENCODING MUTATIONS IN THE HSV-1 ICP27 GENE

**Cells, viruses, infections and transfections.** Infection of cells with viruses and transfection of cells with DNA was carried out in Vero or V27 cells. Vero cells were obtained from the American Type Culture Collection, Rockville, Md.; the derivation of V27 cells is described below. The wild type strain of HSV-1, KOS 1.1 was used in all experiments. Cells were infected at a multiplicity of 10 pfu per cell. Disodium phosphonoacetic acid (PAA) (Abbott Laboratories, North Chicago, I11.) was added to the medium at a concentration of 400 µg/ml as indicated below. Transfection of cells with viral DNA in marker transfer experiments was performed in V27 cells using the calcium phosphate precipitation technique (Rice and Knipe, 1988, J. Virol. 62:3814).

The V27 cell line, which contains a stably integrated copy of the HSV-1 ICP27 gene, was isolated in the following manner. Subconfluent 100mm diameter plates of Vero cells were transfected with 0.8 µg of pSV2neo (Southern and Berg, 1982, J. Mol. Appl. Genet. 1:327), a plasmid conferring resistance to the drug G418, and either 4 or 10 µg of pBH27 (Rice and Knipe, 1988, J. Virol. 62:3814), a plasmid encoding HSV-1 ICP27. Two days later, the cells were passed at a ratio of 1:9 in medium containing 600 µg of G418 per ml. G418-resistant colonies were isolated 2 to 4 weeks later and grown into mass culture. The cell lines were tested for ICP27 expression on the basis of their ability to support plaque formation of the ICP27 mutants *ts*Y46 (Sacks et al., 1985, J. Virol. 55:796) and *ts*LG4 (Sandri-Goldin et al., 1981, J. Virol. 38:41) at the nonpermissive temperature. Six of seventeen isolates tested positive in this assay. One of these cell lines, designated V27, was used for the isolation of ICP27 mutants. Southern blot analysis indicated that V27 cells contained approximately one copy of the ICP27 gene per haploid genome equivalent.
**Generation of HSV-1 ICP27 mutants.** Because ICP27 is an essential gene for the replication of HSV-1 (Sacks et al., 1985, J. Virol. 55:796), viruses containing mutations in ICP27 have a lethal phenotype. V27 cells were therefore used to propagate such mutants.

Insertion of the *E*. *coli lac*Z gene into the HSV-1 chromosome is a useful tool for the isolation of viral mutants (Carmichael and Weller, 1989, J. Virol. 63:591; Goldstein and Weller, 1988, J. Virol. 62:196). Viral plaques expressing β-galactosidase, the product of the *lac*Z gene, can be identified on the basis of their color (blue) in the presence of X-gal, a chromogenic substrate for β-galactosidase. As described in detail below, an HSV-1 mutant expressing β-galactosidase was first isolated which contains an in-frame insertion of the lacZ gene into the ICP27 coding sequences. This virus then served as a recipient in marker transfer experiments for the introduction of specifically mutated ICP27 alleles into the viral genome. Recombinants containing the newly introduced ICP27 genes were identified as clear plaques against a background of parental blue plaques.

In order to make the HSV-1 *lac*Z insertion mutant, a recombinant plasmid was constructed in which the lacZ coding region was inserted into a deleted version of the ICP27 gene. This fusion gene was then cotransfected into V27 cells with infectious HSV-1 DNA. When the progeny viruses from this transfected culture were plated onto V27 cells in the presence of X-gal, approximately 3% of the plaques were blue. A blue plaque was picked, and the resulting virus clone was designated d27-*lac*Z1. Southern blot analysis indicated that d27-*lac*Z1 has a genomic structure consistent with the replacement of the WT ICP27 gene with the ICP27-lacZ fusion gene (Figure 4A). In addition, d27-*lac*Z1-infected cells did not express the WT ICP27 but instead expressed a polypeptide of approximately 137 kDa, consistent with the size predicted for the ICP27-β-galactosidase fusion protein. The stock of d27-*lac*Z1 virus was unable to form plaques on Vero cells (<2 x 10³ pfu/ml) but formed plaques efficiently on V27 cells (2 x 10⁸ pfu/ml). The experimental details for the isolation of d27-*lac*Z1 are now described below.

The plasmid pPs27pd1 (Rice et al., 1989, J. Virol. 63:3399) contains a 6.1-kilobase (kb) PstI insert derived from HSV-1 genomic DNA. This fragment contains the entire ICP27 gene, as well as adjoining sequences (Figure 4A). Derivatives of pPs27pd1 which contain a deletion in the ICP27 gene and and insertion of the *lac*Z gene were constructed in the following manner. pPs27pd1 was linearized in the ICP27 coding region by digestion with SalI. The DNA was then treated with Bal 31 such that approximately 0.5 kb of DNA was removed from each end. The ends were repaired by a fill-in reaction using the Klenow fragment of *E*. *coli* DNA polymerase (Ausubel et al., 1987, Current Protocols in Molecular Biology, John Wiley and Sons, NY). This DNA was ligated to BglII linkers (New England BioLabs, Inc., Beverly, Mass.) and the product was digested with BglII, religated, and used to transform *E. coli.* Four plasmid isolates were obtained each of which contained the *lac*Z gene inserted in the same Orientation as the ICP27 gene.

Each of the four plasmid DNAs was digested with PstI, individually mixed with WT HSV-1 DNA, and transfected into V27 cells. After 4 days, the cultures were harvested and the resulting virus stocks were plated onto V27 cells under a liquid overlay of medium 199 (GIBCO Laboratories, Grand Island, N.Y.) containing 1% newborn calf serum and 0.1% human immunoglobulin. After 2 days, the medium was replaced with medium 199 containing 1% newborn calf serum, 0.5% agarose, and 300 µg of 5-bromo-4-chloro-3-indolyl-β-D-galactopyranoside (X-gal; Boehringer Mannheim Biochemicals, Indianapolis, Ind.) per ml. One of the resulting four stocks of virus gave rise to a high percentage (approximately 3%) of blue plaques. One blue plaque was purified three times, and the resulting virus clone was designated d27-lacZ1. Southern blot analysis of d27-lacZ1 DNA indicated that the WT ICP27 gene had been replaced with the ICP27-*lac*Z fusion gene (Figure 4A). In addition, Southern blot analysis of viral DNA, as well as restriction analysis of the parental plasmid (designated pPsd27-*lac*Z1), indicated that approximately 0.8 kb had been deleted from the ICP27 gene in d27-*lac*Z1.
**Construction of HSV-1 mutants containing deletion or nonsense mutations in the ICP27 gene.** Five plasmids were generated which contained deletions or nonsense codon insertions in the ICP27 gene, as generally described in Knipe et al,. J. Virol. 63:3400 et seq. See Figure 4B. Viral DNA inserts in each plasmid were separated from vector sequences and were cotransfected into V27 cells with d27-*lac*Z1 DNA. Resulting progeny viruses were plated on V27 cells in the presence of X-gal, and a fraction (approximately 1 to 5%) formed clear plaques. Viruses which formed clear plaques were isolated and screened for the presence of the newly introduced ICP27 alleles in an immunofluorescence assay or by DNA restriction analysis as described below. For each mutant, a positive plaque was purified three times. A potential ICP27 deletion mutant was designated d27-1. Potential nonsense mutants were designated n59R, n263R, and n504R; the numbers in the names of the mutants correspond to the number of amino-terminal ICP27 residues expected to be present in each truncated protein. For comparison, the wild type protein consists of 512 amino acid residues.

The recombinant viral genomes were characterized by Southern blot hybridization to confirm that each virus contained the appropriate mutation. Viral DNA was isolated from infected V27 cells and the PstI and XbaI restriction enzyme patterns were examined in Southern blots. A 6.1 kb PstI HSV-1 DNA fragment containing the wild type ICP27 gene was used as a probe. Because this fragment includes some of the repeat sequences in the L component of the HSV-1 genome (Figure 4A), two bands were evident when wild type HSV-1 DNA was examined. These were the 6.1 kb ICP27 fragment and a 3.3 kb fragment which was derived from the other U_{L}-R_{L} junction (Figure 5). In contrast, all five ICP27 mutant DNAs lacked the 6.1 kb fragment but contained the 3.3 kb fragment. The mutant d27-1 contained a new fragment of DNA of approximately 4.6 kb, consistent with its expected 1.6 kb deletion. The four remaining mutants each contained two new bands, the combined sizes of which approximated 6.1 kb, consistent with the insertion of an XbaI site at the appropriate position in each mutant genome. Furthermore, none of the mutant genomes contained the 8.4 kb PstI fragment, which should only be present in the parental d27-*lac*Z1 DNA.

Plaque assays were performed to determine whether the mutants were capable of growth in Vero cells. All five mutants were unable to form plaques on Vero cells at the lowest dilution which could be tested (Table 1) (lower dilutions destroyed the cell monolayer). However, each mutant formed plaques efficiently on V27 cells. Because the only known intact HSV-1 gene resident in the V27 genome is a wild type copy of the ICP27 gene, these results indicate that the lethal defect in each mutant is complemented in trans by this wild type form of ICP27.

**TABLE 1.**

| Growth of HSV-1 ICP27 mutants^{α} | | |
|---|---|---|
| Viral titer (pfu/ml) | | |
| Virus | Vero cells | V27 cells |
| KOS1.1(WT) | 7 x 10⁸ | 5 x 10⁸ |
| d27-1 | <2 x 10³ | 3 x 10⁸ |
| n59R | <2 x 10³ | 3 x 10⁸ |
| n263R | <2 x 10³ | 1 x 10⁸ |
| n406R | <2 x 10³ | 2 x 10⁸ |
| n504R | <2 x 10³ | 3 x 10⁸ |

| | | |
|---|---|---|
| ^{α} Viral stocks were titrated by plaque assay on the cell lines indicated | | |

The experimental details for the isolation of these mutants is now described below.

Deletion and nonsense mutations in the ICP27 gene were engineered into the plasmid pPs27pd1 (Figure 4B). The plasmids containing the 406R and 504R mutations, pPs-406R and pPs-504R respectively, were constructed as described in Rice et al. (1989, J. Virol. 63:3399). The plasmid pPsd27-1 was constructed by digesting pPs27pd1 with BamHI and StuI, filling in the 3' recessed BamHI DNA ends using the Klenow fragment of *E. coli* DNA polymerase, and recircularizing the large DNA fragment with DNA ligase. The plasmids pPs-59R and pPs-263R were constructed by substituting the mutant 2.4 kb BamHI-SstI fragments from the plasmids pBH-59R and pBH-263R described above for the WT 2.4 kb BamHI-SstI fragment of pPs27pd1.

Recombinant viruses were constructed as follows. The plasmid DNAs described above were digested with PstI, individually mixed with d27-*lac*Z1 viral DNA, and transfected into V27 cells. Progeny viruses were harvested 3 to 5 days later and were plated on V27 cells in the presence of X-gal, as described above. Clear plaques, which were observed at frequencies of 0.5 to 5%, were picked and screened to determine whether they had acquired the ICP27 gene mutations.

Plaque isolates were screened in two ways. Plaque isolates d27-1, n59R, and n263R were purified three times in V27 cells and then used to infect V27 cells. Crude viral DNA was prepared from the infected cells (Gao and Knipe, 1989, J. Virol. 63:5258). The XbaI and BamHI restriction enzyme patterns of each DNA sample were examined in order to confirm the presence of each mutation. In the case of n406R and n504R, initial plaque isolates were used to prepare small virus stocks. Vero cells grown on glass cover slips were then infected with each virus. The infected cells were fixed and stained for immunofluorescence using an anti-ICP27 monoclonal antibody, (Ackerman et al., 1984, J. Virol. 52:108). Isolates of n406R and n504R were then plaque purified two more times and large stocks of each mutant were prepared in V27 cells.
**Expression and intracellular localization of mutated ICP27 polypeptides.** The mutants were next examined for expression of ICP27-related polypeptides. Vero cells were either mock-infected or infected with each virus, and cell extracts were prepared at 10 hour PI. Proteins were separated by SDS-PAGE, transferred to nitrocellulose, and reacted with the monoclonal antibody H1113 (Figure 6). No ICP27-related polypeptides were detected in extracts of mock-, d27-1-, or n59R-infected cells. An approximately 38 kDa protein was detected in the extracts of n263-infected cells, and an approximately 52 kDa protein was detected in the extracts of n406-infected cells. Cells infected with n504R produced an ICP27-related polypeptide which comigrated with the 63 kDa wild type protein. The sizes of the truncated proteins were in rough agreement with the predicted sizes based on the DNA sequence of the ICP27 gene (McGeoch et al., 1988, J. Gen. Virol. 69:1531).

To determine the intracellular distribution of the mutant proteins expressed on the viral genome, Vero cells infected with each mutant were harvested at 4 h PI, and were fixed and processed for immunofluorescence microscopy using the monoclonal antibody H1113. Cells infected with the wild type virus exhibited localized nuclear staining (Figure 7C and D) wherein one or more areas stained more intensely. These areas did not correspond to any particular nuclear regions such as nucleoli. No staining above background levels was detected in cells infected with d27-1 or n59R. Cells infected with n263R exhibited nuclear staining and, similar to virus-infected cells, some areas of the nucleus stained more intensely (Figure 7E and F). These areas appeared to correspond to nucleoli. Cells infected with n406R also exhibited nuclear staining, but the pattern of staining differed from that in wild type virus-infected cells in two respects (Figure 7G and H). First, in most cells the n406R encoded ICP27 protein was largely excluded from the nucleolar regions. Second, many n406R-infected cells exhibited a rather punctate pattern of staining, wherein the mutated protein was concentrated in globular clusters in the nucleus. Cells infected with n504R also exhibited nuclear staining, but in this case, the protein appeared to be present throughout the nucleus exhibiting a more diffuse pattern than that seen in wild type virus infected cells. These results indicate that mutated forms of ICP27 encoded by n263R, n406R and n504R efficiently localized to the cell nucleus but differed from each other and from that of wild type virus in their patterns of intranuclear accumulation.
**Viral DNA synthesis in cells infected with ICP27 mutants.** The phenotype of the ICP27 mutants with regard to viral DNA replication was next determined. To determine the viral DNA synthesis phenotype of each mutant, Vero cells were mock-infected or infected with each of the mutants. After a 1 hour adsorption period, the monolayers were washed extensively with warm medium to remove unadsorbed virus. Total DNA was prepared by the method of Challberg (1986, Proc. Natl. Acad. Sci. USA 83:9094) at either 1 or 16 h PI. Purified DNA was quantitated by UV absorption at 260 nm. The DNA was diluted and denatured in 100 mM sodium hydroxide for 30 min at room temperature. An equal volume of 12 x SSC (1 x SSC is 0.15 M sodium chloride plus 0.015 M sodium citrate) was added, and the DNA was applied to a nitrocellulose filter using a slot-blot manifold (Schleicher & Schuell, Keene, N.H.). The filters were baked and the DNA was hybridized to ³²P-labeled HSV-1-specific probes prepared by random primer labeling. Probes included either pSHZ, containing the ICP0 gene (Nabel et al., 1988, Science 239:1299), or pRB3441, containing the gene for Vmw65 (McKnight et al., 1986, Cancer Cells 4:163). An HSV-1 mutant, d102, which contains a large deletion in the ICP8 gene and is therefore unable to replicate its DNA (Gao and Knipe, 1989, J. Virol. 63:5258), was included in these experiments as a negative control. Viral DNA replicated to high levels in wild type virus-infected cells. In contrast, no evidence of viral DNA replication could be detected in d102-infected cells. All five ICP27 mutants were capable of replicating viral DNA during the course of the infection (Figure 8).

To quantitate these results, the amount of radioactivity hybridizing to each slot was measured by scintillation counting and the data are summarized in Table 2. Based on these results, the mutants could be divided into two phenotypic classes with respect to DNA replication. The first class containing mutants d27-1, n59R, n263R, and n406R, exhibited a partial defect in viral DNA amplification (6 to 38% of the wild-type level). The second class, consisting only of n504R, exhibited a wild type phenotype for viral DNA replication. It is important to note that these experiments measured the level of viral DNA accumulation in infected cells, a quantity determined by the rate of DNA synthesis as well as by the stability of the replicated DNA.

**TABLE 2.**

| Viral DNA replication in cells infected with ICP27 mutants | | | | |
|---|---|---|---|---|
| Virus | DNA amplification^{a} in: | | % of wild type amplification^{b}in: | |
| | Expt. 1 | Expt. 2 | Expt. 1 | Expt 2 |
| KOS1.1 (WT) | 80 | 142 | 100 | 100 |
| d102 | 0.5 | 0.4 | | |
| d27-1 | 18 | 11 | 23 | 8 |
| n59R | 15 | 22 | 19 | 15 |
| n263R | 17 | 9 | 21 | 6 |
| n406 | 30 | 23 | 38 | 16 |
| n504 | 98 | 121 | 123 | 85 |

| | | | | |
|---|---|---|---|---|
| ^{a}The numbers shown are the ratio of the amount of HSV-1 DNA in infected Vero cells at 16 h PI to the amount present at 1 h PI. The DNA was detected as shown in Figure 5 and quantitated as described above (experiment 1 corresponds to Figure 5, while experiment 2 was a separate experiment). The probe in experiment 1 was pSHZ, and the probe in experiment 2 was pRB3441. | | | | |
| ^{b}The amount of DNA amplification by the wild type virus in each experiment was normalized to 100%, and the other values are expressed relative to this value. Because d102 exhibited a decrease in the amount of DNA during infection, a percent value was not determined. | | | | |

**Patterns of viral protein synthesis in ICP27 mutant infected cells.** To analyze the effect of mutations in the ICP27 gene on viral gene expression, viral protein synthesis was examined in mutant infected cells. Mock-or HSV-1-infected Vero cells were labeled with 15 µCi [³⁵S]-methionine/ml of medium at 3, 6, or 9 h PI. Proteins were extracted from the cell, separated by SDS-PAGE and visualized by autoradiography (Figure 9). At 3 and 6 h PI, with the exception of n406R, the ICP27 mutants exhibited patterns of protein synthesis that were qualitatively similar to the pattern in wild type virus infected cells. Cells infected with n406R appeared to lack several proteins, including ICP6, ICP8, and the precursor to gB (pgB). However, at 9 h PI, cells infected with all five ICP27 mutants exhibited both quantitative and qualitative differences in viral protein synthesis compared with wild type virus. The five mutants could be divided into four phenotypic classes with respect to viral protein synthesis at 9 h PI. Mutants d27-1 and n59R were reproducibly indistinguishable from each other in terms of their patterns of protein synthesis. Both of these mutants expressed high levels of most β proteins but expressed lower levels (relative to wild type levels), of several γ-1 proteins, including ICP5 and ICP25 (Figure 9, 9 h PI). Mutant n263R exhibited a pattern of protein synthesis at 9 h PI that was very similar to that of d27-1 and n59R, but this mutant expressed slightly more of several γ-1 proteins. Mutant n406R had an unusual phenotype with regard to viral protein synthesis in that greatly reduced levels of many viral proteins including ICP6, ICP8, and pgB were evident in cells infected with this mutant. This effect did not extend to all viral proteins in that higher levels of the γ-1 proteins, ICP5 and ICP25, were evident at 9 hours PI compared with d27-1-infected cells. Mutant n504R expressed high levels of β and γ-1 proteins, such as ICP1/2 and ICP15.

In wild type virus infected cells at 9 hours PI, the expression of the α proteins ICP4 and ICP27, was reduced markedly (Figure 9). This was not the case when cells were infected with any of the five ICP27 mutants. In addition, n504R-infected cells failed to turn off expression of the ICP27 polypeptide (because none of the other mutants encodes a protein that comigrates with WT ICP27, this was the only case in which a direct comparison of ICP27 protein synthesis rates was possible). With the exception of n406R, the ICP27 mutants also exhibited a defect in their ability to negatively modulate the expression of. many β proteins, including ICP6 and ICP8. These results suggest that ICP27 has a negative regulatory effect on the expression of α and β genes.

To determine whether the defect in viral protein synthesis observed in cells infected with mutant viruses could be corrected by expression of the wild type form of the protein, the following experiment was performed. Vero or V27 cells were infected in parallel with wild type virus or with one of the mutants. Infected cell proteins were labeled with [³⁵S]-methionine at 15 h PI and were subsequently analyzed by SDS-PAGE and autoradiography (Figure 10). The pattern of protein expression of each mutant observed at 15 hours PI in Vero cells was similar to that described above for 9 hours PI. However, when V27 cells were infected with each of the mutants, a pattern of protein synthesis more similar to that of the wild type virus was evident.
**Accumulation of viral mRNA in ICP27 mutant virus infected cells.** Steady-state levels of viral mRNAs expressed in mutant infected cells were analyzed by Northern blot hybridization. RNA was isolated from cells infected with each mutant by treating the cells with Nonidet P-40 and was then extracted with phenol-chloroform. It was precipitated in ethanol (Klessig et al., 1975, J. Virol. 16:1850), suspended in buffer, digested with RNase-free DNase I (Bethesda Research Laboratories, Gaithersburg, Md.), extracted with phenol-chloroform and then reprecipitated in ethanol. Ten micrograms of each RNA sample was subjected to electrophoresis through denaturing formaldehyde-agarose gels (Sambrook et al., *Supra*). Following electrophoresis, the RNA was transferred to GeneScreen (Registered Trade Mark) filters (DuPont, NEN Research Products, Boston, Mass.). Hybridization of the RNA to ³²P-labeled probes, was then performed (Rice and Klessig, 1984, J. Virol. 49:35). The probes included pBH27 (encoding the ICP27 gene [Rice and Knipe, cited elsewhere herein), pK1-2 (encoding the ICP4 gene [DeLuca and Schaffer, 1987, Nucl. Acids Res. 15:4491]), and pEcoRI-BamHI-I-I (encoding the gC gene [Frink et al., 1983, J. Virol. 45:634]). Autoradiograms were analyzed densitometrically in an Ultrascan laser densitometer and online integrator (LKB Instruments, Inc., Rockville, Md.).

Specifically, RNA was extracted from Vero cells which were mock-infected, infected with wild type virus or with the mutants n59R, d27-1, or n504R. Wild type virus infections were carried out in the presence or absence of PAA, a specific inhibitor of HSV-1 viral DNA synthesis. Cytoplasmic RNA was isolated from the infected cells at 9 hours PI. Following northern blot transfer, filters were probed with radiolabeled DNAs specific for ICP27 (Figure 11A), ICP4 (Figure 11B), or gC (Figure 11C) mRNA. No ICP27-specific mRNA was evident in mock- or d27-1-infected cells (Figure 11A, lanes 2 and 3). RNA isolated from n59R- or n504R-infected cells (Figure 11A, lanes 4 and 5) contained two- to threefold more 2.0 kb ICP27 mRNA than did RNA obtained from wild type virus-infected cells (Figure 11A, lane 6). This result was qualitatively consistent with the elevated levels of ICP27 protein synthesis observed at 9 h PI in n504R-infected cells.

In contrast to the results obtained with the ICP27-specific mRNA, approximately equal amounts of ICP4 mRNA were observed in d27-1-, n59R-, and wild type virus-infected cells (Figure 11B, lanes 3, 4, and 6), whereas n504R-infected cells accumulated only 1.6-fold more ICP4 mRNA than did wild type virus-infected cells (Figure 11B, lanes 5 and 6). These results were somewhat unexpected because little or no ICP4 protein was evident at 9 hours PI in wild type virus-infected cells. The synthesis of ICP4 was readily detected in cells infected with the ICP27 mutants (Figure 9; 9 h PI). Therefore, the level of expression of ICP4 at 9 hours PI does not reflect the level of cytoplasmic ICP4 transcripts. This suggests that ICP4 mRNA is translated more efficiently in cells infected with ICP27 mutants than in cells infected with wild type virus.

The accumulation of mRNA specific for the γ-2 gene encoding gC was examined in cells infected with the mutants. Inhibition of viral DNA replication by PAA drastically reduced the amount of gC mRNA which accumulated in cells infected with wild type virus (Figure 11C, lanes 1 and 6; gC mRNA could be detected in lane 1 upon longer exposures of the autoradiogram). Neither d27-1-, n59R-, nor n504R-infected cells expressed detectable levels of gC mRNA (Figure 11C, lanes 3, 4, and 5, respectively). This is of particular interest in the case of the mutant n504R, which replicated WT levels of DNA during infection. Expression of γ-2 genes requires both the replication of the viral DNA and a virus-encoded transacting factor, namely ICP27.

### CONSTRUCTION AND CHARACTERIZATION OF MUTANTS IN THE HSV-1 ICP8 GENE

**Isolation of ICP8 expressing cell lines.** Vero cells were transformed with the plasmid pSG18-SacI (Lee and Knipe, 1983, J. Virol. 46:909; Quinlan and Knipe, 1985, Mol. Cell. Biol. 5:957) or p8B-S (Gao et al., 1988, Virology 163:319) and pSVneo (Southern and Berg, 1986, J. Mol. Appl. Genet. 1:327) essentially as described by Deluca et al. (DeLuca et al., 1985, J. Virol. 56:558). After growth in medium containing the antibiotic G418, 21 drug-resistant colonies were isolated, amplified and screened for their ability to complement the growth of the ICP8 mutants *ts*13, *ts*18, and *ts*HA1 (Conley et al., 1981, J. Virol. 37:413; Holland et al., 1984, J. Virol. 49:947). At the nonpermissive temperature, these *ts* mutants formed plaques in 7 of 21 cell lines derived from cultures receiving the ICP8 gene but they did not form plaques in Neo^{r} cells which were derived from cells that were transfected with pSV2neo alone. Cell lines, B10 and S2, derived from a cells transfected with plasmids p8B-S and pSG18-SacI respectively, yielded the highest levels of complementation and were chosen for further study (Table 3). Wild type virus formed plaques in Neo^{r} cells as well as in B10 and S2 cells at both temperatures. The mutant viruses *ts*13, *ts*18 and *ts*HA1 formed plaques efficiently only at 33.5°C in Neo^{r} cells but formed plaques at an efficiency equal to that of wild type at both temperatures in B10 and S2 cells. Southern blot hybridization was performed to determine the copy number of the ICP8 gene in these cell lines, and B10 and S2 cells contained approximately 1 and 10 copies per haploid genome, respectively.

**TABLE 3.**

| Complementation of ICP8 mutants by B10 and S2 cells^{a} | | | | | | |
|---|---|---|---|---|---|---|
| | Titer (10⁹ pfu/ml)^{b} | | | | | |
| Virus | B10 | | S2 | | Neo^{r} | |
| | 33.5-C | 39°C | 33.5°C | 39°C | 33.5°C | 39°C |
| KOS1.1 | 1.7 | 1.7 | 0.7 | 2.0 | 1.3 | 1.8 |
| ts13 | 4.3 | 3.1 | 2.3 | 4.0 | 1.0 | <0.001 |
| ts18 | 3.0 | 3.2 | 2.3 | 2.7 | 1.7 | <0.001 |
| tsHA1 | 2.3 | 3.7 | 3.7 | 1.7 | 2.4 | <0.001 |

| | | | | | | |
|---|---|---|---|---|---|---|
| ^{a}Cultures of B10, S2, and Neo^{r} cells were infected with each virus and incubated at 33.5°C or 39°C. | | | | | | |
| ^{b}Plaque numbers were counted for 2 to 3 days. | | | | | | |

For all experiments, monolayer cultures were infected with wild type or mutant virus at a multiplicity of 20 pfu per cell.
**Plasmids** The plasmids p8B-S, pSV8 and pm1 and their nucleotide numbering system are described (Gao et al., 1988, Virology 163:319; Su and Knipe, 1987, J. Virol. 61:615). The plasmid p8B-S was constructed by cloning a 5.9 kb B*am*HI-*Sac*I fragment (map units 0.374 to 0.411), including the ICP8 promoter, into pUCl8. The plasmid pSV8 was constructed by inserting a 5.5 kb S*ma*I-S*ac*I fragment (map units 0.374 to 0.409) downstream of the simian virus 40 early promoter. The plasmid pm1 was derived from plasmid pSV8 by changing codons 499 and 502 of the ICP8 gene such that they encode cysteine rather than glycine. Mutant ICP8 plasmids used in this study were derived from pICP8 or pSPICP8, in which a 5.5 kb

S*ma*I-S*ac*I fragment (map units 0.374 to 0.409) was inserted into pUC19 or pSP64, respectively. Plasmids pn10 and pn2 were generated by linearization of the plasmid spICP8 (which was achieved by partial digestion with S*ma*I) and subsequent insertion of a 14 nucleotide X*ba*I linker (5'-CTAGTCTAGACTAG-3'; New England BioLabs, Inc., Beverly, Mass.) containing stop codons in all three reading frames at nucleotides 4084 and 3695, respectively. Therefore, pn10 encodes the first 1,160 amino acid residues, and pn2 encodes the first 1,029 amino acid residues of ICP8 as well as 4 additional amino acids, Pro-Ser-Leu-Asp, encoded by the X*ba*I linker sequence. Plasmid pd301 was generated by an internal in-frame deletion of a 2,001-base-pair (bp) N*ot*I fragment (nucleotides 1395 to 3396). Plasmids pd101 and pd102 were constructed as follows: the plasmid pSPICP8 was linearized by partial digestion with S*ma*I, and a 12 nucleotide B*g*lII linker, 5'-GGAAGATCTTCC-3', was ligated to it. A 1,642-bp deletion was generated by digestion with B*g*lII (converted from a S*ma*I site at nucleotide 652) and B*am*HI (nucleotide 2294) to yield plasmid pd101. Thus, pd101 lacks codons for residues 17 to 563 but has an insertion of one Arg codon encoded by the B*gl*II linker sequence. A 1,188 bp deletion was generated by digestion with B*g*lII (converted from S*ma*I at nucleotides 652 and 1840) to yield plasmid pd102. Thus, pd102 lacks codons for residues 17 to 411 of the ICP8 coding sequence but encodes three additional amino acids, Arg-Ser-Ser, in the B*gl*II linker sequence. Both plasmids pd101 and pd102 also contain a 14 nucleotide X*b*aI linker at nucleotide 4419, downstream of the ICP8 poly(A) signal. Because there are other S*ma*I sites around nucleotides 4084 and 1840, both pn10 and pd102 were sequenced to determine the exact mutation sites.
**Construction of mutant viruses.** To examine the functional domains of ICP8, several different types of mutations were introduced into the coding region of the ICP8 gene (Figure 12): (i) nonsense mutations (pn10 and pn2); (ii) internal deletions (pd301, pd101, and pd102); and (iii) a site-specific mutation (pm1) (Gao et al., 1988, Virology 163:319). To facilitate screening for recombinant viruses after marker transfer, a mutant virus containing a *lacZ* gene inserted into the ICP8 coding region was constructed in a manner similar to that described above for the generation of ICP27 mutants. This recombinant virus, designated as HD-2, formed blue plaques in the ICP8-expressing cell lines in the presence of X-Gal, but did not form any plaques in Vero cells. DNA encoding the various mutant forms of ICP8 was recombined into this parental strain, and the resulting progeny were isolated from white plaques. White plaques appeared at frequencies ranging from 2 to 39%. The frequency of white plaques in cells transfected with HD-2 DNA and pd101 or pd102 was not above the background. This was probably due to the limited amount of viral sequences available for recombination between pd101 or pd102 and HD-2 DNA.

The following types of analyses were performed to verify that the recombinant viruses contained the appropriate mutation in the ICP8 gene. Viral DNA was isolated, digested with appropriate restriction enzymes, and analyzed by agarose gel electrophoresis. Southern blot analysis was performed to confirm the presence of mutations in viral DNA and to determine the purity of the mutant virus populations. For example, the 8.2 kb B*am*HI G fragment of wild type DNA (Figure 13, lane 1) was divided into 6.8- and 1.4 kb fragments in *n*2 DNA by digestion with B*am*HI and X*ba*I due to the presence of the X*ba*I linker (lane 3). The junction region of B*am*HI G and V (2.3 kbp) was replaced by the *lac*Z gene in HD-2; therefore, digestion of HD-2 DNA with B*am*HI and X*ba*I generated a 12.6 kb fragment (Figure 13, lane 2). Comparison of K*pn*I digested wild type (Figure 13, lane 4), HD-2 (lane 6), and *n*2 (lane 5) DNAs revealed that wild type and n2 DNAs were similar to each other but differed from that of HD-2 DNA due to the *lac*Z insertion (lane 6).

The experimental details for the isolation of viruses containing mutations in the ICP8 gene are now described below.

The mutant virus HD-2, containing a *lac*Z insertion in the ICP8 gene, was isolated as follows. After deletion of a 780-bp *Xho*I fragment from pICP8, this plasmid was briefly digested with Bal 31, a B*g*lII linker was added to the ends of the DNA, and the *lac*Z gene (Pharmacia, Inc., Piscataway N.J.) was inserted. The *lac*Z gene of pMC1871 contains no transcriptional promoter and also lacks the first eight nonessential amino-terminal codons. The mixture of ICP8:*lac*Z plasmids was transfected into B10 cells along with wild type viral DNA. Progeny virus were isolated and were plated on B10 or S2 cells in medium 199 and 1% calf serum containing 0.1% human immune serum for 1 to 2 days at 37°C. To detect β-galactosidase activity, the medium was then changed to medium 199 plus 1.0% agarose containing 400 µg of 5-bromo-4-chloro-3-indolyl-D-galactopyranoside (X-Gal) per ml, and incubation was continued for 8 to 16 hours. Recombinant viruses were identified as blue plaques and were isolated at a frequency of approximately 0.1 to 0.5%. One mutant isolate, termed HD-2 was plaque purified.

HD-2 served as the parental virus for the generation of all the mutant viruses in this study except for d301. After cotransfection of infectious HD-2 DNA with plasmids encoding a mutated ICP8 gene, recombinant viruses were isolated from white plaques propagated in the presence of X-Gal.

The mutant virus d301 was constructed by cotransfection of B10 cells with infectious wild type viral DNA and the plasmid pd301, in which a 2,001 bp N*ot*I fragment was deleted from the ICP8 coding sequence (Figure 12). Progeny virus from this transfection were tested for their ability to replicated in B10 cells but not in Vero cells.
**Growth properties of mutant viruses.** Each of the mutant viruses presented in Figure 12 was unable to replicate in Vero cells and required complementation by the wild type copy of the ICP8 gene present in B10 or S2 cells. Each of the mutant viruses replicated to levels similar to wild-type levels in these ICP8-expressing cell lines. The sizes of the plaques produced by the mutant viruses were slightly smaller than those produced by wild type virus. Furthermore, in all cases, the mutant viruses maintained their mutant phenotype when propagated in either B10 or S2 cells.
**Expression of ICP8 by mutant viruses.** Viral protein synthesis was examined in mutant infected cells by western blotting as described above. The rabbit polyclonal serum 3-83 (Knipe et al., 1987, J. Virol. 61:276) or the mouse monoclonal antibody 10E-3 (Rose et al., 1986, J. Gen. Virol. 67:1315) was used to detect ICP8 (Figure 14). The sizes of the ICP8 polypeptides specified by the mutant viruses were consistent with the predicted sizes. The mouse monoclonal antibody 10E-3 reacted with ICP8 polypeptides expressed by mutants *pm*1, d101, d102, and d301, but not with those expressed by *n*10 and n2 (Table 4) suggesting that this antibody reacts with an epitope contained, at least in part, within the carboxyl-terminal 36 amino acids of ICP8. These results also indicate that *d*101, *d*102, and *d*301 contain in-frame deletions.

**TABLE 4.**

| Solubility of ICP8 encoded by viral mutants | | | |
|---|---|---|---|
| Virus | % of ICP8 | | Antibody^{b} |
| | Supernatant^{a} | Pellet | |
| KOS1.1 | 81 | 19 | 10E-3 |
| pm1 | 12 | 88 | 10E-3 |
| d101 | 22 | 78 | 10E-3 |
| d102 | 33 | 77 | 10E-3 |
| d301 | 42 | 58 | 10E-3 |
| KOS1.1 | 77 | 23 | 3-83 |
| n10 | 92 | 8 | 3-83 |
| n2 | 10 | 90 | 3-83 |

| | | | |
|---|---|---|---|
| ^{a}The supernatant and pellet fractions were defined as the samples obtained by centrifugation after DNase I treatment (Knipe et al., 1986, J. Virol. 44:736). | | | |
| ^{b}The antibody used for the Western blots to visualize ICP8 was rabbit polyclonal 3-83 (Knipe et al., 1987, J. Virol. 61:276) or mouse monoclonal 10E-3 (Rose et al., 1986, J. Gen. Virol. 67:1315). The negatives of the color reactions of Western blots were scanned by densitometer. | | | |

**Viral DNA replication in mutant virus infected cells.** To examine DNA replication in cells infected with each mutant virus under nonpermissive conditions, Vero cells were infected with each virus and [³H]-thymidine was added to the cultures from 6 to 10 hours PI. The cells were harvested, and the DNA was isolated. Each DNA sample was digested with B*am*HI and X*ho*I and subjected to agarose gel electrophoresis. Each of the mutants was unable to replicate viral DNA (Figure 15, lanes 6 through 11) as was the wild-type virus when grown in the presence of phosphonoacetic acid (Figure 15, lane 5), a compound that preferentially inhibits the HSV-1 DNA polymerase. Wild type levels of DNA replication are shown in Figure 15, lane 4.

The experimental details for the analysis of viral DNA now follow.
(i) Preparation of DNA. Plasmid DNA and viral DNAs were prepared as described by Knipe et al. (1979, J. Virol. 29:698). Viral DNA used for Southern blot analysis was purified as follows. Infected cells at a late times PI were frozen and thawed and then sonicated for 30 s at 0 to 4°C. Cell debris were removed by centrifugation at 480 x g. The resulting supernatant was subjected to centrifugation at 23,500 x g. Pellets were extracted with phenol-chloroform-isoamyl alcohol (24:24:1) three times. After ethanol precipitation, the DNA was dissolved in a Tris-EDTA buffer.
(ii) Measurement of viral DNA synthesis. Cells were infected with the appropriate virus and labeled from 6 to 10 h with 20 µCi of [³H]thymidine per ml, and total DNA was isolated by the method of Challberg (1986, Proc. Natl. Acad. Sci. USA 83:9094). The DNA was digested with the appropriate restriction enzymes and separated by agarose gel electrophoresis. After electrophoresis, the gel was treated with 1.0 M sodium salicylate for fluorography (Chamberlain, 1979, Anal. Biochem. 98:132).
**DNA-binding properties of mutated ICP8.** Prior to examining the DNA-binding properties of mutant ICP8 molecules, the solubility of the individual polypeptides was examined (Table 5) and was found to vary significantly. The DNA-binding properties of the soluble mutated ICP8 molecules were then examined by chromatography through single stranded DNA-cellulose.
Single stranded DNA cellulose chromatography of infected-cell extracts was performed as described (Knipe et al., 1981, J. Virol. 44:736), except that the cells were labeled from 4 to 6 hours PI with [³⁵S]methionine.
Proteins were applied to columns containing single stranded DNA cellulose, and were eluted in increasing concentrations of NaCl. The results of an experiment comparing wild type virus with the mutant *pm*1 are presented in Figure 16. The majority of the ICP8 expressed in wild type virus infected cells bound to the column (compare lanes 4 and 5 in Figure 16A), which was then eluted at an NaCl concentration of 0.5 M (lane 12).
In contrast, very little of the ICP8 expressed in *pm*1 infected cells bound to the column (compare lanes 4 and 5 in Figure 16B). The amount of ICP8 which bound and was then eluted from the column was determined and the results are presented in Table 5. ICP8 expressed in n10 infected cells bound to single stranded DNA-cellulose as efficiently and tightly as did wild-type ICP8. The lowest level of binding (21%) was observed for ICP8 expressed in *d*301 infected cells. ICP8 encoded by amino-terminal deletion mutants *d*101 and *d*102 bound to the DNA cellulose at levels of 72 and 75%, respectively. Based on these results it can be concluded that the portion of ICP8 from amino acid residues 564 to 1160 contains a region required for DNA binding.

**TABLE 5.**

| Ability of mutant ICP8 to bind to single stranded DNA cellulose | | | | | | |
|---|---|---|---|---|---|---|
| % of ICP8: | | | | | | |
| Virus | In flowthrough and wash | Eluted at indicated NaCl concn | | | | Bound |
| | | 0.3M | 0.5M | 1.0M | 4.0M | |
| KOS1.1 | 2 | 23 | 67 | 8 | <1 | 98 |
| pm1 | 69 | 7 | 20 | 4 | <1 | 31 |
| n10 | 2 | 13 | 77 | 8 | <1 | 98 |
| d102 | 25 | 26 | 22 | 20 | 6 | 75 |
| d101^{a} | 28 | 47 | 22 | 5 | <1 | 72 |
| d301^{a} | 79 | 6 | 13 | 2 | <1 | 21 |
| n2^{a} | 54 | 1 | 39 | 3 | <1 | 46 |

| | | | | | | |
|---|---|---|---|---|---|---|
| ^{a}Data obtained from densitometry of the negatives prepared from the Western blots. | | | | | | |

**Nuclear localization of ICP8 molecules encoded by viral mutants.** Wild-type ICP8 localizes to the nucleus efficiently in infected cells (Fenwick et al., 1978, J. Gen. Virol. 39:519; Knipe and Spang, 1982, J. Virol. 43:314; Quinlan and Knipe, 1983, Mol. Cell. Biol. 3:315; Quinlan and Knipe, 1985, Mol. Cell. Biol. 5:957). The cellular distribution of wild-type and mutant ICP8 molecules was examined by indirect immunofluorescence which was performed according to Quinlan and Knipe (1983, Mol. Cell. Biol. 3:315) using a 1:10 dilution of 793 anti-ICP8 monoclonal antibody and a 1:100 dilution of rhodamine-conjugated goat anti-mouse antibody for all mutant viruses except *n*2. A 1:30 dilution of anti-ICSP 11/12 polyclonal serum (Powell et al., 1981, J. Virol. 39:894) and a 1:200 dilution of fluorescein-conjugated goat anti-rabbit immunoglobulin were used for the detection of n2 ICP8. The results are shown in Figure 17. The n10 encoded ICP8 polypeptide, which lacks the last 36 amino acids from the carboxyl terminus and bound to a DNA as efficiently as wild-type ICP8, did not localize to the nucleus, rather it remained in the cytoplasm of infected cells (Figure 17C). In contrast, the *pm*1 ICP8 polypeptide, which bound poorly to DNA, was found predominantly in the nucleus (Figure 17E). These results clearly demonstrate that the nucleus localization signal(s) of ICP8 is separate from the DNA-binding function.

ICP8 encoded by *d*101 localized to the nucleus (Figure 17H) and was also capable of binding to DNA (Table 5), but this virus was incapable of viral DNA replication. The phenotype of this mutant provides genetic evidence that ICP8 has nuclear functions other than binding to DNA.

A summary of the phenotypic properties of the ICP8 mutants is presented below in Table 6.

**TABLE 6.**

| Phenotypic classes of ICP8 mutant viruses | | | | |
|---|---|---|---|---|
| Group | Mutants | Growth on Vero cells | ssDNA binding | Localization |
| A | *n*10,*d*102 | - | + | C |
| B | *pm*1, *d*301 | - | - | N |
| C | *n*2 | - | - | C |
| D | *d*101 | - | + | N |

## Claims

1. A vaccine suspended in a pharmaceutically acceptable carrier comprising a mutated herpesvirus capable of infecting a mammalian cell and eliciting a protective immune response in a mammal vaccinated with said herpesvirus, said herpesvirus being **characterized by** a mutation in a gene encoding at least one of the following proteins: HSV-1 ICP27; HSV-1 ICP8 or homologs thereof, said mutation rendering said virus incapable of replication and wherein said herpesvirus is HSV-1, HSV-2, VZV, EBV, HHV-6 or HHV-7.

2. The vaccine of claim 1, wherein said protein is HSV-1 ICP27 or a homolog thereof and/or said herpesvirus is n504R.

3. The vaccine of claim 1, wherein said protein is HSV-1 ICP8 or a homolog thereof and/or said herpesvirus is d301.

4. The vaccine of claim 1, wherein said herpesvirus further encodes one or more heterologous genes.

5. The vaccine of claim 1, wherein said herpesvirus encodes the wild type form of the viral thymidine kinase gene.

6. A method of making a herpesvirus vaccine, said method comprising suspending a mutated herpesvirus as defined by any one of the claims 1-5, in a pharmaceutically acceptable carrier.

7. The vaccine of any one of claims 1-5 for use in therapy, for example in a method of immunizing a human against a herpesvirus.

8. Use of the vaccine of any one of claims 1-5 for the manufacture of a medicament for use in therapy, for example in a method of immunizing a human against herpesvirus.

9. Use of a herpesvirus as defined by any one of claims 1-5 for the preparation of a vaccine.

## Patentansprüche

1. Ein Impfstoff, suspendiert in einem pharmazeutisch verträglichen Träger, umfassend ein mutiertes Herpesvirus, das in der Lage ist, eine Säugerzelle zu infizieren und eine schützende Immunantwort in einem mit besagtem Herpesvirus geimpften Säuger hervorzurufen, wobei besagtes Herpesvirus, **gekennzeichnet ist durch** eine Mutation in einem Gen codierend zumindest eines der folgenden Proteine: HSV-1 ICP27, HSV-1 ICP8 oder Homologe davon, wobei besagte Mutation besagtes Virus replikationsunfähig macht und worin besagtes Herpesvirus HSV-1, HSV-2, VZV, EBV, HHV-6 oder HHV-7 ist.

2. Der Impfstoff nach Anspruch 1, worin besagtes Protein HSV-1 ICP27 oder ein Homolog davon ist und/oder besagtes Herpesvirus n504R ist.

3. Der Impfstoff nach Anspruch 1, worin besagtes Protein HSV-1 ICP8 oder ein Homolog davon ist und/oder besagtes Herpesvirus d301 ist.

4. Der Impfstoff nach Anspruch 1, worin besagtes Herpesvirus außerdem ein oder mehrere heterologe Gene codiert.

5. Der Impfstoff nach Anspruch 1, worin besagtes Herpesvirus die Wildtyp-Form des viralen Thymidinkinase-Gens codiert.

6. Ein Verfahren zur Herstellung eines Herpesvirus-Impfstoffes, wobei besagtes Verfahren Suspendieren eines mutierten Herpesvirus, wie in einem der Ansprüche 1 bis 5 definiert, in einem pharmazeutisch verträglichen Träger umfasst.

7. Der Impfstoff nach einem der Ansprüche 1 bis 5 zur therapeutischen Verwendung, zum Beispiel in einem Verfahren zur Immunisierung eines Menschen gegen ein Herpesvirus.

8. Verwendung des Impfstoffes nach einem der Ansprüche 1 bis 5 zur Herstellung eines Medikaments zur therapeutischen Verwendung, zum Beispiel in einem Verfahren zur Immunisierung eines Menschen gegen ein Herpesvirus.

9. Verwendung eines Herpesvirus, wie in einem der Ansprüche 1 bis 5 definiert, zur Herstellung eines Impfstoffes.

## Revendications

1. Vaccin en suspension dans un véhicule pharmaceutiquement acceptable, comprenant un herpèsvirus muté capable d'infecter une cellule mammalienne et de susciter une réponse immunitaire protectrice chez un mammifère vacciné avec ledit herpèsvirus, ledit herpèsvirus étant **caractérisé par** une mutation dans un gène codant pour au moins une des protéines suivantes : ICP27 de HSV-1 ; ICP8 de HSV-1 ou des homologues de celles-ci, ladite mutation rendant ledit virus incapable de réplication et dans lequel ledit herpèsvirus est HSV-1, HSV-2, VZV, EBV, HHV-6 ou HHV-7.

2. Vaccin de la revendication 1, dans lequel ladite protéine est ICP27 de HSV-1 ou un homologue de celle-ci et/ou ledit herpèsvirus est n504R.

3. Vaccin de la revendication 1, dans lequel ladite protéine est ICP8 de HSV-1 ou un homologue de celle-ci et/ou ledit herpèsvirus est d301.

4. Vaccin de la revendication 1, dans lequel ledit herpèsvirus code en outre pour un ou plusieurs gènes hétérologues.

5. Vaccin de la revendication 1, dans lequel ledit herpèsvirus code pour la forme de type sauvage du gène viral de thymidine kinase.

6. Procédé de préparation d'un vaccin contre un herpèsvirus, ledit procédé comprenant la mise en suspension, dans un véhicule pharmaceutiquement acceptable, d'un herpèsvirus muté tel que défini par l'une quelconque des revendications 1 à 5.

7. Vaccin de l'une quelconque des revendications 1 à 5, pour utilisation en thérapie, par exemple dans un procédé d'immunisation d'un sujet humain contre un herpèsvirus.

8. Utilisation du vaccin de l'une quelconque des revendications 1 à 5, pour la fabrication d'un médicament pour utilisation en thérapie, par exemple dans un procédé d'immunisation d'un sujet humain contre un herpèsvirus.

9. Utilisation d'un herpèsvirus tel que défini par l'une quelconque des revendications 1 à 5, pour la préparation d'un vaccin.
